# FASCICULE DE BREVET EUROPEEN

(11) **EP 0 847 271 B1**
(45) Date de publication et mention de la délivrance du brevet: **02.05.2002**
(21) Numéro de dépôt: 97928344.7
(22) Date de dépôt: 12.06.1997
(51) Int. Cl.: A61K 7/13, C07D 487/04

(54) **DERIVES DE PYRAZOLO-(1,5-a)-PYRIMIDINE, LEUR PREPARATION ET TEINTURES DE FIBRES KERATINIQUES LES CONTENANT**
DERIVATE DES PYRAZOLO-(1,5-a)-PYRIMIDINE, DESSEN HERSTELLUNG UND HAARFÄRBEMITTEL MIT SOLCHEN DERIVATEN
PYRAZOLO-(1,5-a)-PYRIMIDINE DERIVATIVES, THEIR PREPARATION AND HAIR DYES CONTAINING THESE

(30) Priorité: 21.06.1996 FR 9607776
(43) Date de publication de la demande: 17.06.1998
(73) Titulaire: L'OREAL, 75008 Paris (FR)
(72) Inventeur: TERRANOVA, Eric, F-92600 Asnières (FR); FADLI, Aziz, F-93150 Le Blanc Mesnil (FR); LAGRANGE, Alain, F-77450 Coupvray (FR)
(74) Mandataire: Miszputen, Laurent
(86) Numéro de dépôt international: FR9701057
(87) Numéro de publication internationale: WO9749378

(56) Documents cités:
- EP-A- 0 433 854
- CHEMICAL ABSTRACTS, vol. 67, no. 3, 17 juillet 1967 Columbus, Ohio, US; abstract no. 11498c, page 1097; XP002029042 & JP 06 703 174 A (SHIONOGI)
- W.E. KIRKPATRICK ET AL.: "3-Halo-5,7-dimethylpyrazolo[1,5-a]pyrimid ines" JOURNAL OF MEDICINAL CHEMISTRY, vol. 77, no. 3, 1977, pages 386-393, XP002029041

## Description

L'invention a pour objet de nouvelles compositions pour la teinture d'oxydation des fibres kératiniques comprenant au moins un dérivé pyrazolo-[1,5-a]-pyrimidine à titre de base d'oxydation, le procédé de teinture mettant en oeuvre cette composition, de nouveaux dérivés pyrazolo-[1,5-a]-pyrimidine ainsi que leur procédé de préparation.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine. Ils doivent également présenter une bonne stabilité chimique dans les formulations. Ils doivent présenter un bon profil toxicologique.

Il a déjà été proposé, notamment dans la demande de brevet DE 4 029 324, d'utiliser certains dérivés de pyrazolo-[1,5-a]-pyrimidine, pouvant être substitués par des radicaux alkyles en C₁-C₄ en position 4, 5 et/ou 6, comme coupleurs pour la teinture d'oxydation des fibres kératiniques.

Il a été proposé aussi dans la demande de brevet DE 4 133 957, d'utiliser certains dérivés de pyrazolo-[1,5-a]-pyrimidine appartenant à la famille des tétrahydro pyrazolo-[1,5-a]-pyrimidine comme précurseurs de colorant d'oxydation pour la teinture d'oxydation des fibres kératiniques.

La demanderesse vient maintenant de découvrir, de façon totalement inattendue et surprenante, une nouvelle famille de dérivés de pyrazolo-[1,5-a]-pyrimidine de formule (I) définie ci-après, pour partie nouveaux en sol, pouvant convenir pour une utilisation comme précurseurs de colorant d'oxydation, mais en outre permettant d'obtenir des compositions tinctoriales qui conduisent à des colorations puissantes et qui présentent une bonne tenue face aux agents extérieurs (lumière, intempéries, lavage, ondulation permanente, transpiration, frottements). Enfin, ces composés s'avèrent être aisément synthétisables et sont chimiquement stables. Ils présentent un bon profil toxicologique.

Ces découvertes sont à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture, au moins un dérivé de pyrazolo-[1,5-a]-pyrimidine de formule (I) ci-dessous à titre de base d'oxydation et/ou un de ses sels d'addition avec un acide ou avec une base et/ou une de ses formes tautomères, lorsqu'il existe un équilibre tautomérique : 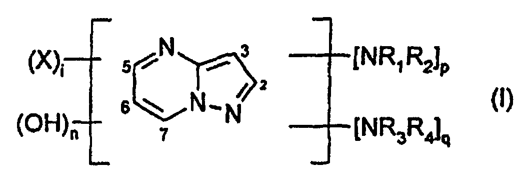 dans laquelle :
- R₁, R₂ R₃ et R₄ désignent , identiques ou différents un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical amino alkyle en C₁-C₄ (l'amine pouvant être protégée par un acétyle, un uréido, un sulfonyl), un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl- ou di-[hydroxy(C₁-C₄) alkyl]-amino alkyle en C₁-C₄ ;
- les radicaux X désignent, identiques ou différents, un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl ou di-[hydroxy(C₁-C₄)alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;
- i vaut 0, 1, 2 ou 3 ;
- p vaut 0 ou 1 ;
- q vaut 0 ou 1 ;
- n vaut 0 ou 1 ;
sous réserve que :
- (i) la somme p + q est différente de 0 ;
- (ii) lorsque p + q est égal à 2, alors n vaut 0 et les groupes NR₁R₂ et NR₃R₄ occupent les positions (2,3) ; (5,6) ; (6,7) ; (3,5) ou (3,7) ;
- (iii) lorsque p + q est égal à 1 alors n vaut 1 et le groupe NR₁R₂ (ou NR₃R₄) et le groupe OH occupent les positions (2,3) ; (5,6) ; (6,7) ; (3,5) ou (3,7).

Lorsque les composés de formule (I) sont tels qu'ils comportent un groupe OH sur l'une des positions 2, 5 ou 7 en α d'un atome d'azote, il existe un équilibre tautomérique représenté par exemple par le schéma suivant :

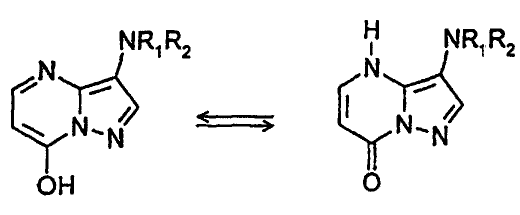

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates. Les sels d'addition avec une base utilisables dans le cadre des compositions tinctoriales de l'invention (bases d'oxydation et coupleurs) sont notamment ceux obtenus avec la soude, la potasse, l'ammoniaque ou les amines.

Parmi les dérivés de pyrazolo-[1,5-a]-pyrimidine de formule (I), utilisables à titre de base d'oxydation dans les compositions conformes à l'invention, on peut notamment citer :
- la pyrazolo-[1,5-a]-pyrimidine-3,7-diamine ;
- la 2-méthyl pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol ;
- le 3-amino 5-méthyl pyrazolo-[1,5-a]-pyrimidin-7-ol ;
- le 3-amino pyrazolo-[1,5-a]-pyrimidin-5-ol ;
- le 2-(3-amino pyrazolo-[1,5-a]-pyrimidin-7-ylamino)-éthanol ;
- la 3-amino-7-β-hydroxyéthylamino-5-méthyl-pyrazolo-[1,5-a]-pyrimidine;
- le 2-(7-amino pyrazolo-[1,5-a]-pyrimidin-3-ylamino)-éthanol ;
- le 2-[(3-amino-pyrazolo-[1,5-a]-pyrimidin-7-yl)-(2-hydroxyéthyl)-amino]-éthanol ;
- le 2-[(7-amino-pyrazolo-[1,5-a]-pyrimidin-3-yl)-(2-hydroxyéthyl)-amino]-éthanol ;
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N-7, N-7-tetraméthyl pyrazolo-[1,5-a]-pyrimidine-3,7-diamine ;
et leurs sels d'addition et leurs formes tautomères, lorsqu'il existe un équilibre tautomérique.

Les dérivés de pyrazolo-[1,5-a]-pyrimidine de l'invention de formule (I) peuvent être préparés selon des méthodes connues et décrites dans la littérature. On pourra se reporter à titre d'exemples aux références suivantes:

Les dérivés de pyrazolo-[1,5-a]-pyrimidine de l'invention de formule (I) peuvent être préparés par cyclisation à partir d'un aminopyrazole selon les synthèses décrites dans les références suivantes :
- EP 628559 BEIERSDORF-LILLY
- R. Vishdu, H. Navedul, *Indian J. Chem.,* 34b (6), 514, 1995.
- N.S. Ibrahim, K.U. Sadek, F.A. Abdel-Al, *Arch. Pharm.,* 320, 240, 1987.
- R.H. Springer, M.B. Scholten, D.E. O'Brien, T. Novinson, J.P. Miller, R.K. Robins, *J*.
   *Med. Chem.,* 25, 235, 1982.
- T. Novinson, R.K. Robins, T.R. Matthews, *J. Med. Chem*., 20, 296, 1977.
- US 3907799 ICN PHARMACEUTICALS

Les dérivés de pyrazolo-[1,5-a]-pyrimidine de formule (I) de l'invention peuvent être préparés par cyclisation à partir d'hydrazine selon les synthèses décrites dans les références suivantes :
- A. McKillop et R.J. Kobilecki, *Heterocycles*, 6(9), 1355, 1977.
- E. Alcade, J. De Mendoza, J.M. Marcia-Marquina, C. Almera, J. Elguero, *J. Heterocyclic Chem.,* 11(3), 423, 1974.
- K. Saito, I. Hori, M. Higarashi, H. Midorikawa, *Bull. Chem. Soc. Japan,* 47(2), 476, 1974.

A titre d'illustration, les dérivés de 3-amino pyrazolo-[1,5-a]-pyrimidine de formule (I) de l'invention peuvent, par exemple, être préparés suivant le procédé décrit au schéma 1.

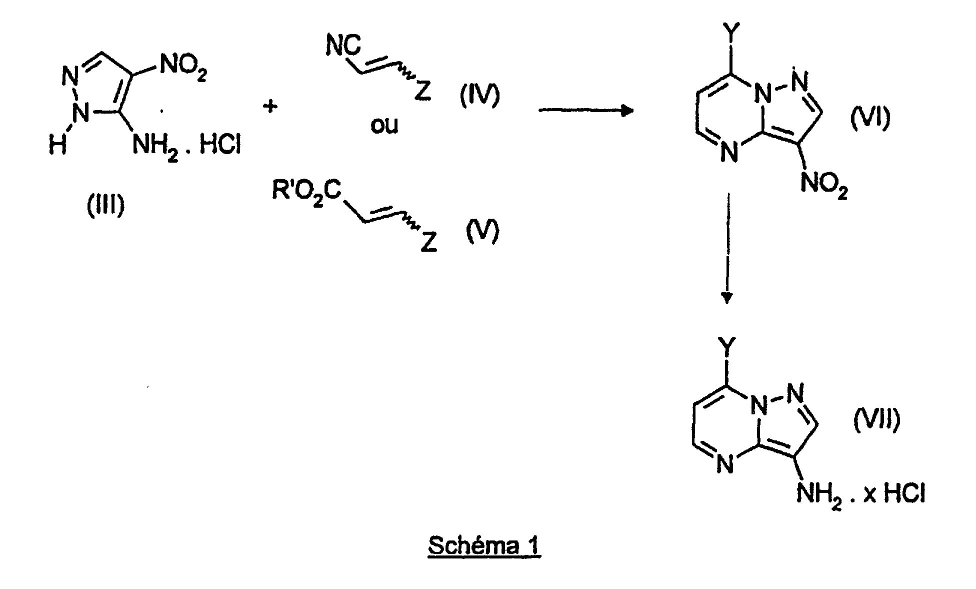

Le chlorhydrate de 4-nitro-2H-pyrazol-3-ylamine (III) (préparé selon H. Dorn et H. Dilcher, *Liebigs Ann.Chem.,* 707, 141, 1967) peut être cyclisé en présence d'un dérivé d'acrylonitrile (IV) (Z=MeO, EtO ou Me₂N) ou d'un acrylate (V) (Z=MeO, EtO ou Me₂N ; R'= alkyle C₁-C₄, aryle) pour conduire aux pyrazolo-[1,5-a]-pyrimidines de structure (VI) (Y=NH₂,OH). Cette réaction peut être faite en s'inspirant de la méthode de G. Mühmel, R. Hanke et E. Breitmaier décrite dans *Synthesis,* 673, 1982. La liste des dérivés qui peuvent être cyclisés avec le 4-nitro-2H-pyrazol-3-ylamine (III) n'est pas limitée aux seuls dérivés d'acrylonitrile et d'acrylate. On peut citer par exemple les dérivés de β-céto ester (VIII) (X possède la même définition que pour les X de la formule précédente (I) ; R' = alkyle C₁-C₄, aryle), de β-céto nitrile (IX) (X possède la même définition que pour les X de la formule précédente (I)) ou encore de β-cyano acétal (X) (R'= alkyle C₁-C₄) sans être limitatif.

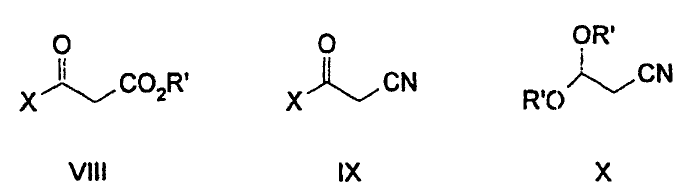

Les pyrazolo-[1,5-a]-pyrimidines de structure (VI) peuvent être ensuite réduits selon des procédés connus (R. Hemmer, W. Lürken, dans *Houben-Weyl,* "Methoden der Organischen Chemie", vol.E16d, p 815ff.). On préférera utiliser des métaux comme le palladium (Pd), le platine (Pt) ou le nickel (Ni) en présence de donneur d'hydrogène comme le formiate d'ammonium, l'acide formique ou encore le cyclohexène à la place de l'hydrogène (S.Ram, R.E. Ehrenkaufer, *Synthesis,* 91, 1988). On pourra également utiliser des métaux comme le zinc (Zn), l'étain (Sn) ou le fer (Fe) en milieu acide tel que l'acide chlorhydrique aqueux ou l'acide acétique aqueux, éventuellement avec addition d'un solvant organique comme le méthanol, l'éthanol ou le tétrahydrofuranne.

Le ou les dérivés de pyrazolo-[1,5-a]-pyrimidine de formule (I) ci-dessus représentent de préférence de 0,0005 à 12 % en poids environ du poids total de la composition tinctoriale, et encore plus préférentiellement de 0,005 à 6 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 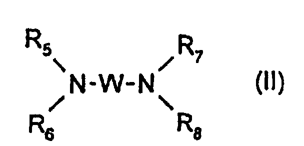 dans laquelle W est un reste propylène éventuellement substitué par un groupement hydroxyle ou un radical alkyle en C₁-C₄ ; R₅, R₆, R₇ et R₈, identiques ou différents, représentent un atome d'hydrogène, un radical alkyle en C₁-C₄ ou hydroxyalkyle en C₁-C₄.

La composition tinctoriale conforme à l'invention peut encore contenir, en plus des colorants définis ci-dessus, au moins une base d'oxydation additionnelle qui peut être choisie parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénols et des bases hétérocycliques différentes des dérivés de pyrazolo-[1,5-a]-pyrimidine de formule (I) utilisés conformément à l'invention.

Parmi les paraphénylènediamines, on peut plus particulièrement citer à titre d'exemple, la paraphénylènediamine, la paratoluylènediamine, la 2,6-diméthyl paraphénylènediamine, la 2-β-hydroxyéthyl paraphénylènediamine, la 2-n-propyl paraphénylènediamine, la 2-isopropyl paraphénylénediamine, la N-(β-hydroxypropyl) paraphénylènediamine, la N,N-bis-(β-hydroxyéthyl) paraphénylènediamine, la 4-amino N-(β-méthoxyéthyl) aniline, les paraphénylènediamines décrites dans la demande de brevet français FR 2 630 438, et leurs sels d'addition.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-amino phényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition .

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxy-méthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition .

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut plus particulièrement citer à titre d'exemple, les dérivés pyridiniques, les dérivés pyrimidiniques, les dérivés pyrazoliques différents des dérivés de pyrazolo-[1,5-a]-pyrimidine de formule (I) utilisés conformément à l'invention, et leurs sets d'addition.

Lorsqu'elles sont utilisées, ces bases d'oxydation additionnelles représentent de préférence de 0,0005 à 12 % en poids environ du poids total de la composition tinctoriale, et encore plus préférentiellement de 0,005 à 6 % en poids environ de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques et leurs sels d'addition .

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthyl)amino 1-méthoxy benzène, le 1,3-diamino benzène, le 1,3-bis-(2,4-diaminophénoxy) propane, la 3-uréido aniline, le 3-uréido 1-diméthylamino benzène, le sésamol, l'α-naphtol, le 6-hydroxy indole, le 4-hydroxy indole, le 4-hydroxy N-méthyl indole, et leurs sels d'addition.

Lorsqu'ils sont présents ces coupleurs représentent de préférence de 0,0001 à 10 % en poids environ du poids total de la composition tinctoriale et encore plus préférentiellement de 0,005 à 5 % en poids environ de ce poids.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogénes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, pendant un temps suffisant pour développer la coloration désirée, soit à l'air, soit à l'aide d'un agent oxydant. La composition tinctoriale peut éventuellement contenir des catalyseurs d'oxydation, afin d'accélérer le processus d'oxydation.

Selon une première forme de mise en oeuvre du procédé de l'invention, la coloration des fibres peut être effectuée sans addition d'un agent oxydant, au seul contact de l'oxygène de l'air.

Selon une deuxième forme de mise en oeuvre du procédé de l'invention, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon cette deuxième forme de mise en oeuvre du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, aprés quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Certains composés de formule (I), utilisés à titre de base d'oxydation dans le cadre de la présente invention, sont nouveaux et, à ce titre, constituent un autre objet de l'invention.

Ces nouveaux dérivés de pyrazolo-[1,5-a]-pyrimidine, leurs sels d'addition avec un acide ou une base et leurs formes tautomères lorsqu'il existe un équilibre tautomérique répondent à la formule (I') suivante : 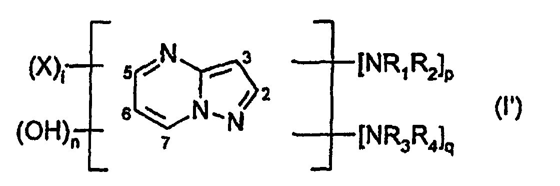 dans laquelle les radicaux R₁, R₂, R₃, R₄, X, i, n, p et q ont les mêmes significations que celles indiquées précédemment dans la formule (I), à l'exception des composés suivants :
- la pyrazolo-[1,5-a]-pyrimidine-6,7-diamine ;
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N 7, N 7-tétraméthyl pyrazolo-[1,5-a]-pyrimidine-3,7-diamine ;
- la 2,3-diméthyl pyrazolo-[1,5-a]-pyrimidine-6,7-diamine ;
- le 6-amino-5-méthyl-pyrazolo-[1,5-a]-pyrimidin-7-ol ;
- la 2,5-diméthyl 6-phényl-[1,5-a]-pyrimidine-3,7-diamine ;
- la 2,6-diméthyl 5-benzyl-[1,5-a]-pyrimidine-3,7-diamine ;
et de leurs sels d'addition.

Parmi les nouveaux composés de formule (I'), on peut notamment citer :
- la pyrazolo-[1,5-a]-pyrimidine-3,7-diamine;
- la 2-méthyl pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol ;
- le 3-amino 5-méthyl pyrazolo-[1,5-a]-pyrimidin-7-ol ;
- le 3-amino pyrazolo-[1,5-a]-pyrimidin-5-ol ;
- le 2-(3-amino pyrazolo-[1,5-a]-pyrimidin-7-ylamino)-éthanol ;
- la 3-amino-7-β-hydroxyéthylamino-5-méthyl-pyrazolo-[1,5-a]-pyrimidine;
- le 2-(7-amino pyrazolo-[1,5-a]-pyrimidin-3-ylamino)-éthanol ;
- le 2-[(3-amino-pyrazolo-[1,5-a]-pyrimidin-7-yl)-(2-hydroxyéthyl)-amino]-éthanol ;
- le 2-[(7-amino-pyrazolo-[1,5-a]-pyrimidin-3-yl)-(2-hydroxyéthyl)-amino]-éthanol ;
ainsi que leurs sels d'addition et leurs formes tautomères lorsqu'il existe un équilibre tautomérique.

Les dérivés de pyrazolo-[1,5]-pyrimidine de formule (I) ainsi que leurs sels d'addition et leurs formes tautomères tels que définis ci-dessus peuvent également être utilisés comme base d'oxydation dans et pour la préparation de compositions destinées à la photographie ou à l'imagerie chimique.

Les exemples qui suivent sont destinés à illustrer l'invention sans pour autant en limiter la portée.

### EXEMPLE 1: DICHLORHYDRATE DE PYRAZOLO-[1,5-a]-PYRIMIDINE-3,7-DIAMINE

### 1^{ère} étape : CHLORHYDRATE DE 3-NITRO-PYRAZOLO-[1,5-a]-PYRIMIDIN-7-YLAMINE

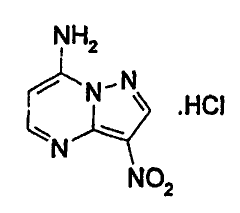

On a introduit dans un ballon tricol de 500 cc muni d'une agitation mécanique et équipé d'un réfrigérant et d'un thermomètre, 50 g de chlorhydrate de 4-nitro-2H-pyrazol-3-ylamine (préparé selon H. Dorn et H. Dilcher, Liebigs Ann.Chem., 707, 141, 1967), 35 g de β-éthoxy acrylonitrile et 250 cc d'acide acétique. On a porté le milieu au reflux pendant 4h30'. On a refroidit vers 40°C puis on a filtré le précipité. On l'a repris sous agitation dans 300 cc d'éther éthylique. On a filtré à nouveau le précipité, on l'a lavé sur le filtre avec 100 cc d'éther éthylique et on l'a séché sous vide et sur anhydride phosphorique. On a obtenu 61,3 g de chlorhydrate de 3-nitro-pyrazolo-[1,5-a]-pyrimidin-7-ylamine sous forme de poudre jaune. (Rendement = 93 %)
RMN (DMSO d6) : 6,70 (d ; 1H) ; 8,34 (d ; 1H) ; 8,99 (s ; 1H) ; 9,56 (s ; NH₂) ; 11,96 (s ; NH⁺)
ANALYSE ELEMENTAIRE : C₆ H₅ N₅ O₂. HCI PM=215,6

| | **C** | **H** | **N** |
|---|---|---|---|
| Calculée (%) | 33.43 | 2.81 | 32.48 |
| Trouvée (%) | 34.09 | 2.89 | 32.53 |

### 2^{ème} étape : DICHLORHYDRATE DE PYRAZOLO-[1,5-a]-PYRIMIDIN-3,7-DIAMINE

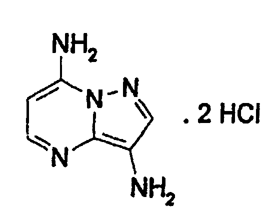

On a introduit dans un ballon tricol de 1000 cc muni d'une agitation magnétique et équipé d'un réfrigérant et d'un thermomètre, 30 g de chlorhydrate de 3-nitro-pyrazolo-[1,5-a]-pyrimidin-7-ylamine, 7 g de palladium sur charbon à 10%, 85 g de cyclohexène et 600 cc d'acide acétique. On a porté le milieu au reflux pendant 4h30', puis on a filtré le catalyseur sur célite. On a repris ce catalyseur imprégné de produit au reflux de 500 cc d'eau et on l'a filtré de nouveau. Les deux filtrats ont été réunis et évaporés. On a obtenu 40 g de poudre beige. Ce solide a été repris dans 55 cc d'acide chlorhydrique concentré et porté au reflux pendant 3h. Le produit a été filtré à 15°C et séché sous vide et sur anhydride phosphorique. On a obtenu 25 g de poudre blanc cassé qu'on a recristallisé dans 80 cc d'acide chlorhydrique concentré. On a recueilli 18 g de dichlorhydrate de pyrazolo-[1,5-a]-pyrimidine-3,7-diamine sous forme de poudre blanche. (rendement=60%).
RMN (DMSO d6) : 6,45 (d ; 1H) ; 8,36 (d ; 1H) ; 8,39 (s ; 1H) ; 8,60-11,50 (6H)
ANALYSE ELEMENTAIRE : C₆ H₇ N₅. 2 HCl . 0,5 H₂O PM=231

| | **C** | **H** | **N** |
|---|---|---|---|
| Calculée (%) | 31.15 | 4.32 | 30,29 |
| Trouvée (%) | 31.12 | 4.29 | 30.34 |

### EXEMPLE 2: CHLORHYDRATE DE 3-AMINO-PYRAZOLO-[1,5-a]-PYRIMIDIN-7-OL

### 1^{ère} étape : 3-NITRO-PYRAZOLO-[1,5-a]-PYRIMIDIN-7-OL

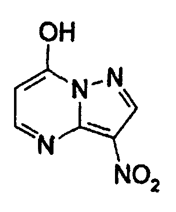

On a introduit dans un ballon tricol de 50 cc muni d'une agitation magnétique et équipé d'un réfrigérant et d'un thermomètre, 2 g de chlorhydrate de 4-nitro-2H-pyrazol-3-ylamine (préparé selon H. Dorn et H. Dilcher, Liebigs Ann.Chem., 707, 141, 1967), 1,55 g de 3-méthoxy acrylate de méthyle et 20 cc d'éthanol absolu. On a porté le milieu au reflux pendant 5 h, puis on a filtré le précipité à chaud. On a obtenu 1,2 g de solide jaune. Après chromatographie sur gel de silice (MERCK: 230-400 mesh ; AcOEt/MeOH=9/1), on a recueilli 0,4 g de 3-nitro-pyrazolo-[1,5-a]-pyrimidin-7-ol sous forme de poudre jaune.
(Rendement = 18%).
RMN (DMSO d6) : 6,19 (d ; 1H) ; 7,98 (d ; 1H) ; 8,75 (s ; 1H) ; 13,10 (OH)

### 2^{ème} étape : CHLORHYDRATE DE 3-AMINO-PYRAZOLO-[1,5-a]-PYRIMIDIN-7-OL

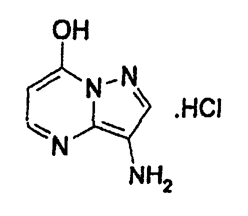

On a introduit dans un ballon tricol de 50 cc muni d'une agitation magnétique et équipé d'un réfrigérant et d'un thermomètre, 0,35 g de 3-nitro-pyrazolo-[1,5-a]-pyrimidin-7-ol, 20 cc d'acide acétique, 1,6 g de cyclohexène et 85 mg de palladium à 10%. On a porté le milieu au reflux pendant 1h30', puis on a filtré le catalyseur sur célite. Après évaporation de l'acide acétique, on a repris le solide obtenu au reflux de 2 cc d'acide chlorhydrique concentré pendant 2h30'. Après évaporation du solvant, on a recueilli un solide blanc cassé.
RMN (D₂O) : 5,93 (d ; 1H) ; 7,87 (d ; 1H) ; 8,04 (s ; 1H)

### EXEMPLE 3: CHLORHYDRATE DE 3-AMINO-5-METHYL-PYRAZOLO-[1,5-a]-PYRIMIDIN-7-OL

### 1^{ère} étape : 3-NITRO-5-METHYL-PYRAZOLO-[1,5-a]-PYRIMIDIN-7-OL

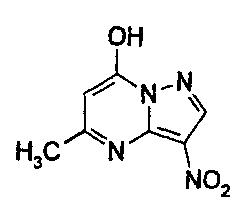

On a introduit, dans un ballon tricol de 500 cc équipé d'une agitation magnétique, d'un thermomètre et d'un réfrigérant, 50 g de chlorhydrate de 4-nitro-2H-pyrazol-3-ylamine (préparé selon H. Dorn et H. Dilcher, Liebigs Ann.Chem., 707, 141, 1967) et 60 g d'acéto-acétate d'éthyle dans 160 cc d'acide acétique. On a porté le milieu réactionnel au reflux pendant 12 h. On a filtré vers 90°C le précipité qui s'est formé. On l'a rincé à l'éther diisopropylique et on l'a séché sous vide sur anhydride phosphorique. On a obtenu 50 g de 3-nitro-5-méthyl-pyrazolo-[1,5-a]-pyrimidin-7-ol sous forme de cristaux jaunes (Rendement = 84,5 % ; Point de fusion = 290°C avec décomposition).
RMN (DMSO d6) : 2,42 (s, 3H) ; 6,03 (s, 1H) ; 8,61 (d, 1H) 12,69 (s, 1H)
ANALYSE ELEMENTAIRE : C₇H₆N₄O₃ PM = 194,15

| | **C** | **H** | **N** | **O** |
|---|---|---|---|---|
| Calculée (%) | 43,31 | 3,12 | 28,86 | 24,72 |
| Trouvée (%) | 43,12 | 3,11 | 28,77 | 24,65 |

### 2^{ème} étape : CHLORHYDRATE DE 3-AMINO-5-METHYL-PYRAZOLO-[1,5-a]-PYRIMIDIN-7-OL

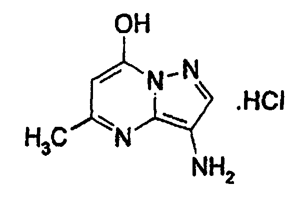

Dans un autoclave de 1 litre, on a introduit 150 cc d'acide acétique et 150 cc d'eau, puis 10 g de 3-nitro-5-méthyl-pyrazolo-[1,5-a]-pyrimidin-7-ol et 1 g de Palladium sur charbon à 5% contenant 50 % d'humidité (ENGELHARD). On a introduit 5 bars d'hydrogène dans le réacteur préchauffé à 30°C. Après 1h de réaction, on a filtré le catalyseur sur célite. Le filtrat a été acidifié avec 100 cc d'une solution d'acide chlorhydrique 7M. Le chlorhydrate précipite sous agitation. On l'a filtré et lavé à l'ether diisopropylique. On a obtenu 4,2 g de chlorhydrate de 3-amino-5-méthyl-pyrazolo-[1,5-a]-pyrimidin-7-ol sous forme de cristaux blancs. (Rendement=41%)
RMN (DMSO d6) : 2,37 (s, 3H) ; 5,71 (s, 1H) ; 8,00 (s, 1H) ; 10,32 (s élargi, 3H) ; 13,09 (s élargi, 1H)
ANALYSE ELEMENTAIRE : C₇ H₈ N₄ O . HCl PM=200,63

| | **C** | **H** | **N** | **O** | **Cl** |
|---|---|---|---|---|---|
| Calculée (%) | 41,91 | 4,52 | 27,93 | 7,97 | 17,67 |
| Trouvée (%) | 41,43 | 4,57 | 27,69 | 8,90 | 17,66 |

### EXEMPLE 4: DICHLORHYDRATE DE 3-AMINO-7-β-HYDROXYETHYL-AMINO-5-METHYL-PYRAZOLO-[1,5-a]-PYRIMIDINE

### 1^{ère} étape : 7-CHLORO-5-METHYL-3-NITRO-PYRAZOLO-[1,5-a]-PYRIMIDINE

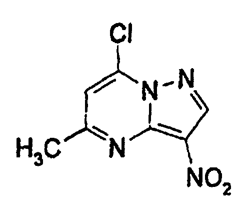

Dans un tricol de 500 cc équipé d'une agitation magnétique, d'un thermomètre et d'un réfrigérant, on a introduit 230 cc d'oxychlorure de phosphore, 15,4 g de N,N-diméthyl aniline et 23,3 g de 3-amino-5-méthyl-pyrazolo-[1,5-a]-pyrimidin-7-ol. Le milieu réactionnel a été porté au reflux pendant 2h30. Après évaporation de l'oxychlorure de phosphore sous pression réduite, on a obtenu une huile verte très visqueuse à laquelle on a ajouté environ 400 g de glace. Un solide brun a précipité. Après 30 minutes d'agitation, on l'a filtré et rincé à l'éther de pétrole puis à l'éther diisopropylique. Après séchage sous vide sur anhydride phosphorique, on a obtenu 21,4 g de 7-chloro-5-méthyl-3-nitro-pyrazolo-[1,5-a]-pyrimidine sous forme d'un solide brun (Rendement = 83,9 %).
RMN (DMSO d6) : 2,70 (s, 3H) ; 7,82 (s, 1H) ; 9,10 (s, 1H)

### 2^{ème} étape : 7-β-HYDROXYETHYLAMINO-5-METHYL-3-NITRO-PYRAZOLO-[1,5-a]-PYRIMIDINE

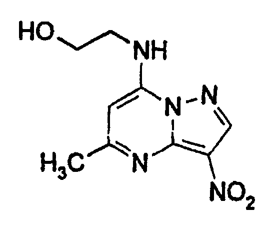

Dans un ballon tricol de 250 cc équipé d'une agitation magnétique, d'un thermomètre et d'un réfrigérant, on a introduit 15 g de 7-chloro-5-méthyl-3-nitro-pyrazolo-[1,5-a]-pyrimidine dans 100 cc d'éthanol. On a additionné goutte à goutte 5 g d'éthanolamine et on a porté le milieu au reflux pendant 30 minutes. Après refroidissement à température ambiante, on a filtré le précipité jaune. On l'a rincé à l'ether diisopropylique. Après séchage sous vide et sur anhydride phosphorique, on a obtenu 14,2 g de 7-β-hydroxyéthylamino-5-méthyl-3-nitro-pyrazolo-[1,5-a]-pyrimidine sous forme de cristaux jaunes.(Rendement=86 %, Point de fusion = 231°C).
RMN (DMSO d6) : 2,52 (s, 3H) ; 3,52 (m, 2H) ; 3,66 (m, 2H) ; 4,96 (t, 1H) ; 6,64 (s, 1H) ; 8,48 (t,1H) ; 8,89 (s, 1H)
ANALYSE ELEMENTAIRE: C₉ H₁₁ N₅ O₃ PM=237,22

| | **C** | **H** | **N** | **O** |
|---|---|---|---|---|
| Calculée (%) | 45,57 | 4,67 | 29,52 | 20,23 |
| Trouvée (%) | 45,09 | 4,59 | 29,40 | 20,63 |

### 3^{ème} étape : DICHLORHYDRATE DE 3-AMINO-7-β-HYDROXYETHYL-AMINO-5-METHYL-PYRAZOLO-[1,5-a]-PYRIMIDINE

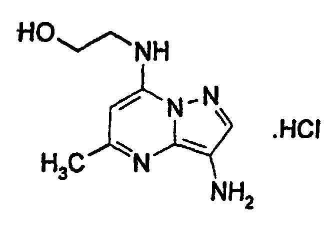

Dans un autoclave de 500 cc, on a introduit 14 de 7-β-hydroxyéthylamino-5-méthyl-3-nitro-pyrazolo-[1,5-a]-pyrimidine dans 150 cc d'acide acétique et 150 cc d'eau, puis 1 g de Palladium sur charbon à 5% contenant 50 % d'humidité (ENGELHARD). On a préchauffé le milieu réactionnel à 30°C et on a introduit 8 bars de pression d'hydrogène. La réaction a débuté aussitôt et la température a atteint 60°C. En fin de réaction, on a filtré le catalyseur sur célite. Le filtrat a été acidifié avec une solution d'acide chlorhydrique 7M. Le chlorhydrate a précipité sous agitation. On l'a filtré et lavé à l'ether diisopropylique. On a obtenu 10 g dichlorhydrate de 3-amino-7-β-hydroxyéthylamino-5-méthyl-pyrazolo-[1,5-a]-pyrimidine sous forme de cristaux légèrement gris. (Rendement = 60 %)
RMN (D₂O) : 2,73 (s, 3H) ; 3,91 (m, 2H) ; 3,98 (m, 2H) ; 6,66 (s, 1H) ; 8,39 (s, 1H)
ANALYSE ELEMENTAIRE: C₉ H₁₃ N₅ O . 2 HCl PM=280,16

| | **C** | **H** | **N** | **O** | **Cl** |
|---|---|---|---|---|---|
| Calculée (%) | 38,59 | 5,4 | 25,05 | 5,71 | 25,31 |
| Trouvée (%) | 38,52 | 5,32 | 24,59 | 6,42 | 25,15 |

### EXEMPLE 5: DICHLORHYDRATE DE 2-METHYL-PYRAZOLO-[1,5-a]-PYRIMIDIN-3,7-DIAMINE

### 1^{ère} étape : CHLORHYDRATE DE 2-METHYL-PYRAZOLO-[1,5-a]-PYRIMIDIN-7-YLAMINE

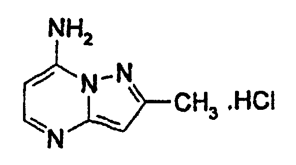

Dans un tricol de 500 cc équipé d'une agitation magnétique, d'un thermomètre et d'un réfrigérant, on a introduit 150 cc d'acide chlorhydrique à 35 % et on a additionné goutte à goutte 47,5 g de 3-amino-5-méthylpyrazole en solution dans 100 cc d'eau. La température est montée à 60°C. On a ensuite additionné 47,5 g de 3-éthoxyacrylonitrile et on a porté la réaction au reflux pendant 1h. On a refroidit le milieu réactionnel et on l'a concentré sous pression réduite. On a rajouté 50 cc d'acétone et on a filtré le précipité obtenu. On l'a rincé à l'éther diisopropylique. Après séchage sous vide sur anhydride phosphorique, on a obtenu 78,7 g de chlorhydrate de 2-méthyl-pyrazolo-[1,5-a]-pyrimidin-7-ylamine sous forme de cristaux blancs (Rendement = 83 %).
RMN (DMSO d6) : 2,43 (s, 3H) ; 6,42 (s, 1H) ; 6,46 (d, 1H) ; 8,26 (d, 1H); 9,55 (s élargi, 1H) ; 10,32 (s élargi, 1H)
ANALYSE ELEMENTAIRE: C₇ H₈ N₄. HCl. 0,5 H₂O PM=193,63

| | **C** | **H** | **N** | **O** | **Cl** |
|---|---|---|---|---|---|
| Calculée (%) | 43,42 | 5,20 | 28,93 | 4,13 | 18,30 |
| Trouvée (%) | 43,68 | 5,20 | 28,83 | 4,66 | 18,58 |

### 2^{ème} étape : 2-METHYL-3-NITRO-PYRAZOLO-[1,5-a]-PYRIMIDIN-7-YLAMINE

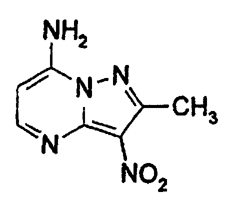

Dans un tricol de 100 cc équipé d'une agitation magnétique, d'un thermomètre et d'un réfrigérant, on a introduit 27 cc d'acide sulfurique à 98% puis on a dissout 5,5 g de chlorhydrate de 2-méthyl-pyrazolo-[1,5-a]-pyrimidin-7-ylamine par petites portions à 5°C. On a ensuite additionné goutte à goutte, en 30 minutes, un mélange de 1,98 g d'acide nitrique fumant et 5 cc d'acide sulfurique à 98%. Après 2h30' de réaction, on a versé le milieu sur 200 cc d'eau glacée et on a neutralisé avec 122 g d'ammoniaque à 20%. On a filtré le précipité vert qui s'est formé. Après séchage sous vide sur anhydride phosphorique, on a obtenu 3,8 g de 2-méthyl-3-nitro-pyrazolo-[1,5-a]-pyrimidin-7-ylamine sous forme d'une poudre verte (Rendement brut = 66%).
RMN (DMSO d6) : 2,62 (s, 3H) ; 6,39 (d, 1H) ; 8,24 (d, 1H) ; 8,39 (s élargi, 2H)

### 3^{ème} étape : DICHLORHYDRATE DE 2-METHYL-PYRAZOLO-[1,5-a]-PYRIMIDIN-3,7-DIAMINE

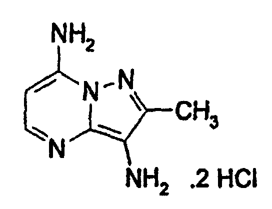

Dans un réacteur de 250 cc, on a introduit 3,9 g de 2-méthyl-3-nitro-pyrazolo-[1,5-a]-pyrimidin-7-ylamine dans 150 cc de méthanol puis 0,42 g de Palladium sur charbon à 5% contenant 50 % d'humidité (ENGELHARD). On a introduit 10 bars de pression d'hydrogène dans le réacteur et on a port le milieu à 90°C. Après 40 minutes de réaction, on a filtré le catalyseur sur célite et on a fait passer un courant d'acide chlorhydrique gazeux à travers le filtrat. Après 1h d'agitation, on a filtré le précipité. On l'a lavé à l'ether diisopropylique et on l'a séché sous vide sur anhydride phosphorique. On a obtenu 2,2 g de dichlorhydrate de 2-méthyl-pyrazolo-[1,5-a]-pyrimidin-3,7-diamine sous forme de cristaux gris (Rendement = 46,5%).
RMN (DMSO d6) : 2,60 (s, 3H) ; 6,50 (d, 1H) ; 8,45 (d, 1H) ; 9,98 (s élargi, 2H) ; 10,88 (s élargi, 4H)
ANALYSE ELEMENTAIRE: C₇ H₉ N₅ . 2HCl PM=236,1

| | **C** | **H** | **N** | **Cl** |
|---|---|---|---|---|
| Calculée (%) | 35,61 | 4,70 | 29,66 | 30,03 |
| Trouvée (%) | 35,16 | 4,85 | 29,32 | 29,79 |
| Calculée avec 0,16 mole d'H₂O | 35,18 | 4,77 | 29,30 | 29,66 |

### EXEMPLES D'APPLICATION

### EXEMPLES 1 A 9 DE TEINTURE EN MILIEU ALCALIN

On a préparé les compositions tinctoriales, conformes à l'invention, suivantes (teneurs en grammes) :

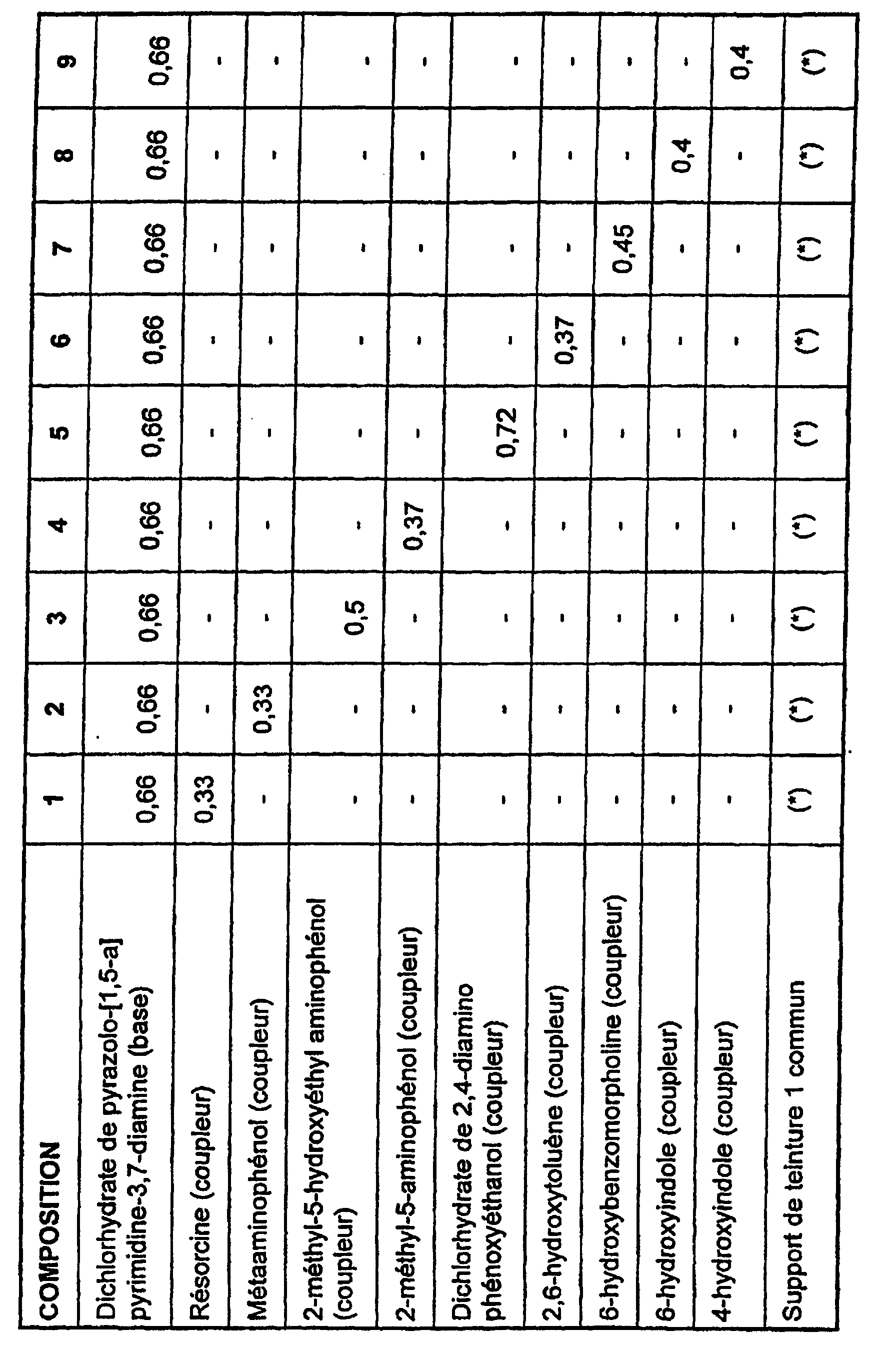

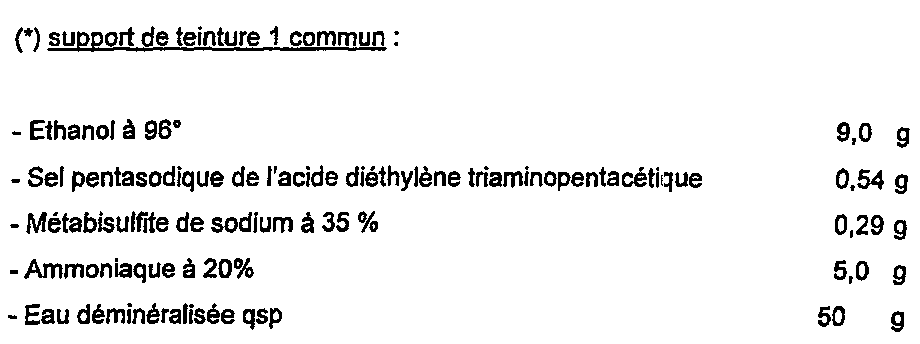

Au moment de l'emploi, on a mélangé chacune des compositions tinctoriales 1 à 9 avec une quantité de 50 g d'une solution de peroxyde d'hydrogène à 20 volumes (6 % en poids) dont le pH a été ajusté à environ 2,5 avec de l'acide orthophosphorique.

Chaque composition résultante a été appliquée immédiatement pendant 30 minutes sur des mèches de cheveux gris naturels à 90 % de blancs ou permanentés, à raison de 10 g pour 1 g de cheveux. Les mèches de cheveux ont ensuite été rincées, lavées avec un shampooing standard puis séchées.

Les mèches de cheveux ont été teintes dans les nuances figurant dans le tableau ci-dessous :

| **EXEMPLE** | **pH DE TEINTURE** | **RESULTAT COLORIMETRIQUE** |
|---|---|---|
| **1** | 10 ± 0,2 | Marron cuivré |
| **2** | 10 ± 0,2 | Irisé rouge |
| **3** | 10 ± 0,2 | Irisé cuivré |
| **4** | 10 ± 0,2 | irisé cuivré |
| **5** | 10 ± 0,2 | Violine irisé |
| **6** | 10 ± 0,2 | Cuivré irisé |
| **7** | 10 ± 0,2 | Irisé rouge |
| **8** | 10 ± 0,2 | Marron cendré |
| **9** | 10 ± 0,2 | Violine |

### EXEMPLES 10 à 18 DE TEINTURE EN MILIEU ACIDE

On a préparé les compositions tinctoriales, conformes à l'invention, suivantes (teneurs en grammes) :

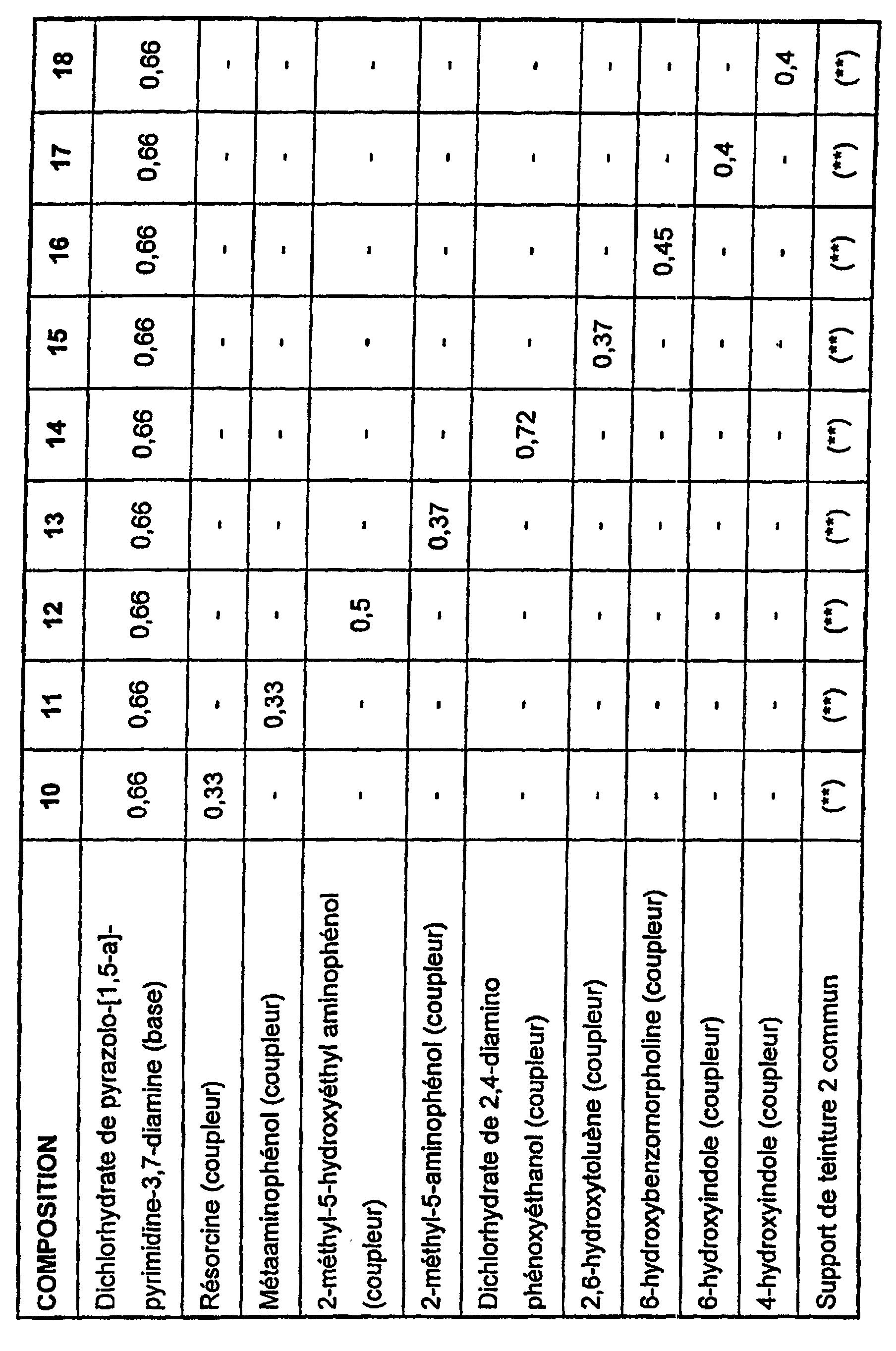

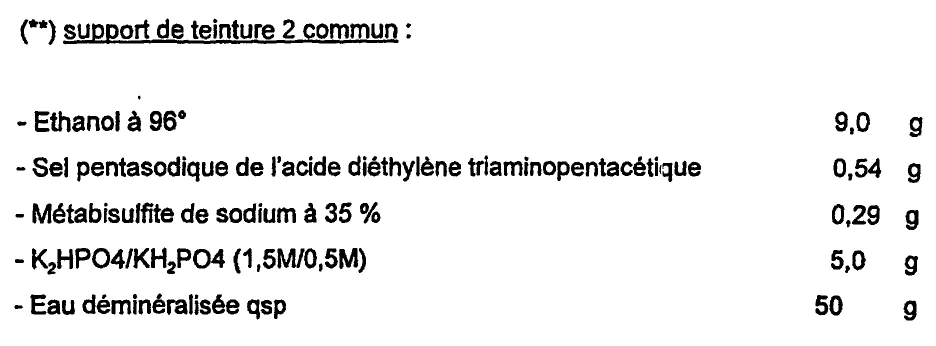

Au moment de l'emploi, on a mélangé chacune des compositions tinctoriales 10 à 18 avec une quantité de 50 g d'une solution de peroxyde d'hydrogène à 20 volumes (6 % en poids) dont le pH a été ajusté à environ 2,5 avec de l'acide orthophosphorique.

Chaque composition résultante a été appliquée immédiatement pendant 30 minutes sur des mèches de cheveux gris naturels à 90 % de blancs ou permanentés, à raison de 10 g pour 1 g de cheveux. Les mèches de cheveux ont ensuite été rincées, lavées avec un shampooing standard puis séchées.

Les mèches de cheveux ont été teintes dans les nuances figurant dans le tableau ci-dessous :

| **EXEMPLE** | **pH DE TEINTURE** | **RESULTAT COLORIMETRIQUE** |
|---|---|---|
| **10** | 6,2 ± 0,2 | Marron cuivré |
| **11** | 5,5 ± 0,2 | Irisé rouge |
| **12** | 6 ± 0,2 | Irisé cuivré |
| **13** | 6,1 ± 0,2 | Irisé cuivré |
| **14** | 5,9 ± 0,2 | Violine irisé |
| **15** | 5,3 ± 0,2 | Cuivré irisé |
| **16** | 5,8 ± 0,2 | Irisé rouge |
| **17** | 5,9 ± 0,2 | Marron cendré |
| **18** | 5,9 ± 0,2 | Violine |

### EXEMPLES 19 A 21 DE TEINTURE EN MILIEU ALCALIN

On a préparé les compositions tinctoriales, conformes à l'invention, suivantes (teneurs en grammes) :

| **COMPOSITION** | **19** | **20** | **21** |
|---|---|---|---|
| Dichlorhydrate de pyrazolo--[1,5-a]pyrimidine-3,7-diamine (base) | 0,66 | 0,66 | - |
| Chlorhydrate de 3-amino 5-méthyl pyrazolo-[1,5-a]-pyrimidin-7-ol (base) | - | - | 0,796 |
| Dichlorhydrate de 3-uréido aniline (coupleur) | 0,67 | - | |
| 3-uréido 1-diméthylamino benzène (coupleur) | - | 0,54 | |
| Dichlorhydrate de 2,4-diaminophénoxyéthanol | - | - | 0,72 |
| Support de teinture 1 commun | (*) | (*) | (*) |

| | | | |
|---|---|---|---|
| (*) Support de teinture 1 commun : Il est identique à celui utilisé dans les exemples 1 à 9 ci-dessus. | | | |

Les teintures ont ensuite été réalisées selon le procédé décrit précédemment pour les exemples 1 à 9 ci-dessus.

Les mèches de cheveux ont été teintes dans les nuances figurant dans le tableau ci-dessous :

| **EXEMPLE** | **pH DE TEINTURE** | **RESULTAT COLORIMETRIQUE** |
|---|---|---|
| **19** | 10 ± 0,2 | Bleu violine |
| **20** | 10 ± 0,2 | violine |
| **21** | 9.7 ± 0,2 | Aubergine |

### EXEMPLES 22 A 24 DE TEINTURE EN MILIEU ACIDE

On a préparé les compositions tinctoriales, conformes à l'invention, suivantes (teneurs en grammes) :

| **COMPOSITION** | **22** | **23** | **24** |
|---|---|---|---|
| Dichlorhydrate de pyrazolo-[1,5-a] pyrimidine-3,7-diamine(base) | 0,66 | 0,66 | - |
| Chlorhydrate de 3-amino 5-méthyl pyrazolo-[1,5-a]-pyrimidin-7-ol (base) | - | - | 0,796 |
| Dichlorhydrate de 3-uréido aniline (coupleur) | 0,67 | - | |
| 3-uréido 1-diméthylamino benzène (coupleur) | - | 0,54 | |
| Dichlorhydrate de 2,4-diaminophénoxyéthanol | - | - | 0,72 |
| Support de teinture 2 commun | (**) | (**) | (**) |

| | | | |
|---|---|---|---|
| (**) Support de teinture 2 commun : Il est identique à celui utilisé dans les exemples 10 à 18 ci-dessus. | | | |

Les teintures ont ensuite été réalisées selon le procédé décrit précédemment pour les exemples 10 à 18 ci-dessus.

Les mèches de cheveux ont été teintes dans les nuances figurant dans le tableau ci-dessous :

| **EXEMPLE** | **pH DE TEINTURE** | **RESULTAT COLORIMETRIQUE** |
|---|---|---|
| **22** | 6 ± 0,2 | Bleu violine |
| **23** | 6.2 ± 0,2 | violine |
| **24** | 5.3 ± 0,2 | Aubergine |

### EXEMPLES COMPARATIFS 25 à 32

On a préparé les compositions tinctoriales, conformes à l'invention, suivantes (teneurs en grammes) :

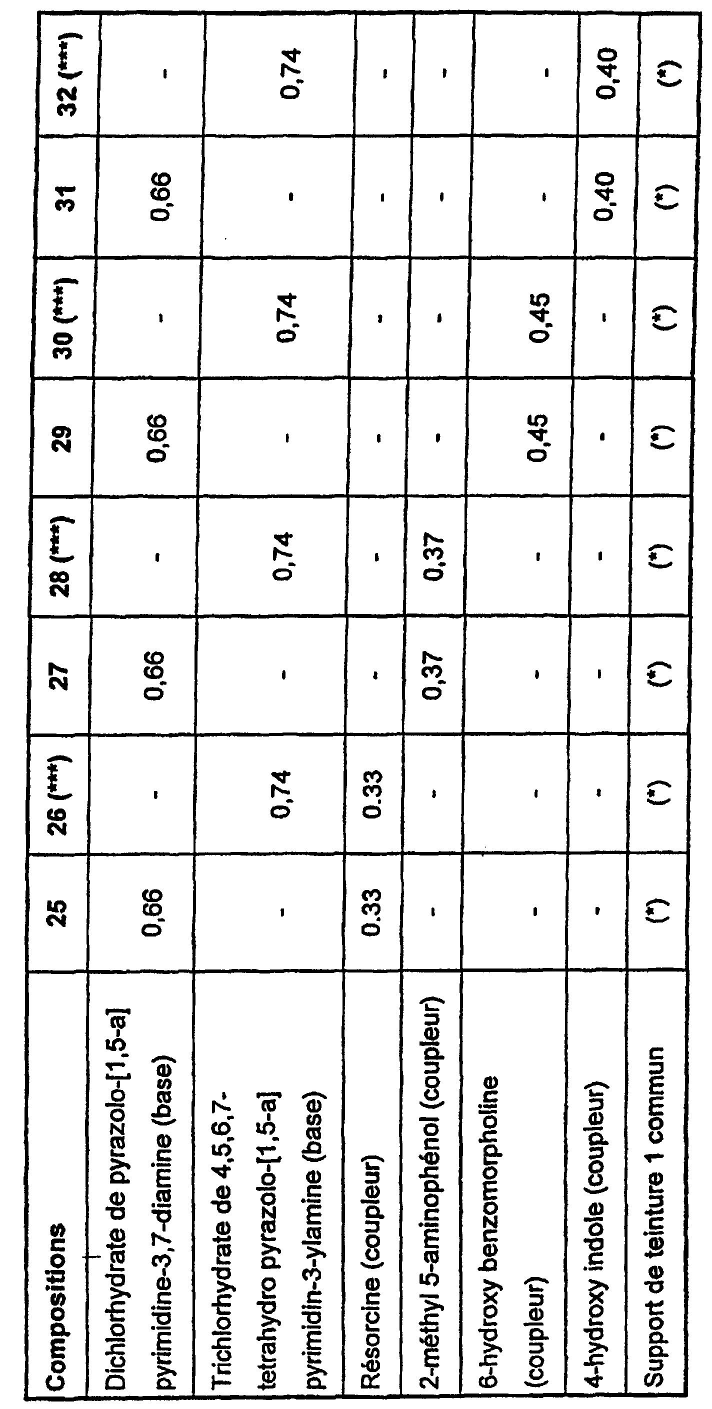

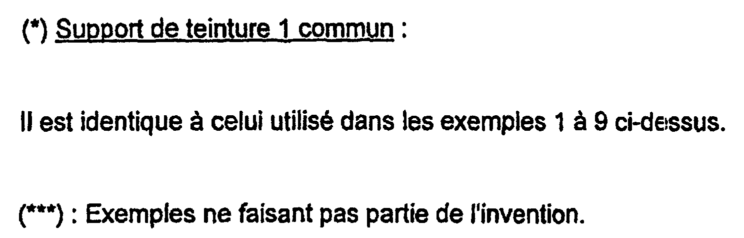

Les teintures ont ensuite été réalisées sur des mèches de cheveux gris naturels à 90 % de blancs, selon le procédé décrit précédemment pour les exemples 1 à 9 ci-dessus.

La couleur des mèches a ensuite été évaluée dans le système MUNSELL au moyen d'un colorimètre CM 2002 MINOLTA.

Les mèches de cheveux ainsi teintes ont ensuite été soumises à un test de résistance aux shampooings (machine automatique).

Pour ce faire, les mèches de cheveux ont été placées dans un godet que l'on a immergé dans une solution d'un shampooing standard à 37°C. Le panier a été soumis à un mouvement de va-et-vient vertical de fréquence variable ainsi qu'à un mouvement de rotation qui reproduisent l'action d'un frottement manuel, ce qui engendre la formation de mousse.

Après 3 minutes d'épreuve, on a retiré les mèches que l'on a rincées puis séchées. Les mèches teintes ont été soumises à 6 épreuves de shampooing consécutives.

La couleur des mèches a été ensuite évaluée à nouveau dans le système MUNSELL au moyen d'un colorimètre CM 2002 MINOLTA de façon à déterminer la dégradation des colorations après ces 6 shampooings.

Selon la notation MUNSELL, une couleur est définie par l'expression **H V / C** dans laquelle les trois paramètres désignent respectivement la teinte ou Hue (**H**), l'intensité ou Value (**V**) et la pureté ou Chromaticité (**C**), la barre oblique de cette expression est simplement une convention et n'indique pas un ratio.

La différence de couleur entre deux mèches est calculée en appliquant la formule de NICKERSON : ΔE = 0,4 CoΔH + 6ΔV + 3 ΔC, telle que décrite par exemple dans "Couleur, Industrie et Technique" ; pages 14-17 ; vol. n° 5 ; 1978.

Dans cette formule, ΔE représente la différence de couleur entre deux mèches, ΔH, ΔV et ΔC représentent la variation en valeur absolue des paramètres H, V et C et Co représente la pureté de la mèche par rapport à laquelle on désire évaluer la différence de couleur.

Les résultats sont donnés dans le tableau ci-dessous :

| **EXEMPLE** | **Couleur des cheveux avant les shampooings** | **Couleur des cheveux après les shampooings** | **Dégradation de la couleur** | | | |
|---|---|---|---|---|---|---|
| | | | **ΔH** | **ΔV** | **ΔC** | **ΔE** |
| **25** | 1.8 YR 3.2/3.7 | 1.4 YR 3.5/2.9 | 0.4 | 0.3 | 0.8 | **4.8** |
| **26** (***) | 6.2 RP 3.1/4.7 | 7.1 RP 3.3/2.7 | 0.9 | 0,2 | 2.0 | **8.9** |
| **27** | 8.9 R 4.0/4.8 | 8.6 R 4.4/4.1 | 0.3 | 0.4 | 0.7 | **5.1** |
| **28** (***) | 4.3 R 2.7/6.2 | 2.9 R 3.3/6.0 | 1.4 | 0.6 | 0.2 | **7.7** |
| **29** | 4.7 R 2.8/4.8 | 3.6 R 3.2/4.6 | 1.1 | 0.4 | 0.2 | **5.1** |
| **30** (***) | 4.5 RP 2.4/3.1 | 3.5 RP 3.1/2.6 | 1.0 | 0.7 | 0.5 | **6.9** |
| **31** | 4.3 RP 2.6/3.9 | 3.7 RP 2.8/2.9 | 0.6 | 0,2 | 1.0 | **5.1** |
| **32**(***) | 8.4 P 2.0/4.4 | 7.4 P 2.6/5.1 | 1.0 | 0.6 | 0.7 | **7.5** |

| | | | | | | |
|---|---|---|---|---|---|---|
| (***) : Exemples ne faisant pas partie de l'invention. | | | | | | |

Ces résultats montrent que les compositions des exemples 25, 27, 29 et 31 conformes à l'invention, c'est à dire contenant du dichlorhydrate de pyrazolo-[1,5-a] pyrimidine-3,7-diamine à titre de base d'oxydation, conduisent à une coloration résistant mieux aux shampooings que les compositions des exemples 26, 28, 30 et 32 ne faisant pas partie de l'invention, c'est à dire contenant du trichlorhydrate de 4,5,6,7-tetrahydro pyrazolo-[1,5-a] pyrimidin-3-ylamine à titre de base d'oxydation, tel que décrit par exemple dans la demande de brevet allemand DE 4 133 957.

## Revendications

1. Composition pour la teinture d'oxydation des fibres kératiniques et en particulier des fibres kératiniques humaines telles que les cheveux, **caractérisée par le fait qu'**elle comprend, dans un milieu approprié pour la teinture, au moins un dérivé de pyrazolo-[1,5-a]-pyrimidine de formule (I) ci-dessous à titre de base d'oxydation et/ou un de ses sels d'addition avec un acide ou une base et/ou une de ses formes tautomères lorsqu'il existe un équilibre tautomérique : 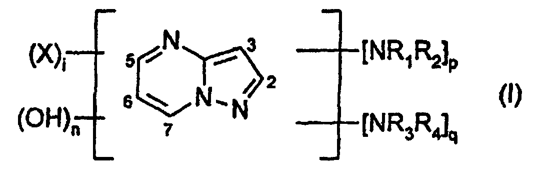 dans laquelle :
- R₁, R₂ R₃ et R₄ désignent, identiques ou différents un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical amino alkyle en C₁-C₄ (l'amine pouvant être protégée par un acétyle, un uréido, un sulfonyl), un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl- ou di-[hydroxy(C₁-C₄) alkyl]-amino alkyle en C₁-C₄ ;
- les radicaux X désignent , identiques ou différents, un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C4)alkyl ou di-[hydroxy(C₁-C₄)alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;
- i vaut 0, 1, 2 ou 3 ;
- p vaut 0 ou 1 ;
- q vaut 0 ou 1 ;
- n vaut 0 ou 1 ;
sous réserve que :
- (i) la somme p + q est différente de 0 ;
- (ii) lorsque p + q est égal à 2 alors n vaut 0 et les groupes NR₁R₂ et NR₃R₄ occupent les positions (2,3) ; (5,6) ; (6,7) ; (3,5) ou (3,7) ;
- (iii) et lorsque p + q est égal à 1 alors n vaut 1 et le groupe NR₁R₂ (ou NR₃R₄) et le groupe OH occupent les positions (2,3) ; (5,6) ; (6,7) ; (3,5) ou (3,7).

2. Composition selon la revendication 1, **caractérisée par le fait que** les dérivés de pyrazolo-[1,5-a]-pyrimidine de formule (I) sont choisis parmi :
- la pyrazolo-[1,5-a]-pyrimidine-3,7-diamine ;
- la 2-méthyl pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol ;
- le 3-amino 5-méthyl pyrazolo-[1,5-a]-pyrimidin-7-ol ;
- le 3-amino pyrazolo-[1,5-a]-pyrimidin-5-ol ;
- le 2-(3-amino pyrazolo-[1,5-a]-pyrimidin-7-ylamino)-éthanol ;
- la 3-amino-7-β-hydroxyéthylamino-5-méthyl-pyrazolo-[1,5-a]-pyrimidine;
- le 2-(7-amino pyrazolo-[1,5-a]-pyrimidin-3-ylamino)-éthanol ;
- le 2-[(3-amino pyrazolo-[1,5-a]-pyrimidin-7-yl)-(2-hydroxyéthyl)-amino]-éthanol ;
- le 2-[(7-amino pyrazolo-[1,5-a]-pyrimidin-3-yl)-(2-hydroxyéthyl)-amino]-éthanol ;
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N-7, N-7-tetraméthyl pyrazolo-[1,5-a]-pyrimidine-3,7-diamine ;
et leurs sels d'addition et leurs formes tautomères, lorsqu'il existe un équilibre tautomérique.

3. Composition selon l'une quelconque des revendications précédentes, **caractérisée par le fait que** le ou les dérivés de pyrazolo-[1,5-a]-pyrimidine de formule (I) représentent de 0,0005 à 12 % en poids du poids total de la composition tinctoriale.

4. Composition selon la revendication 3, **caractérisée par le fait que** le ou les dérivés de pyrazolo-[1,5-a]-pyrimidine de formule (I) représentent de 0,005 à 6 % en poids du poids total de la composition tinctoriale.

5. Composition selon l'une quelconque des revendications précédentes, **caractérisée par le fait que** le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

6. Composition selon l'une quelconque des revendications précédentes, **caractérisée par le fait qu'**elle présente un pH compris entre 3 et 12.

7. Composition selon l'une quelconque des revendications précédentes, **caractérisée par le fait qu'**elle renferme au moins une base d'oxydation additionnelle choisie parmi les paraphénylènediamines, les bis-phénylalkylènediamines, les para-aminophénols, les ortho-aminophénols et des bases hétérocycliques différentes des dérivés de pyrazolo-[1,5-a]-pyrimidine de formule (I).

8. Composition selon la revendication 7, **caractérisée par le fait que** la ou les bases d'oxydation additionnelles représentent de 0,0005 à 12 % en poids du poids total de la composition tinctoriale.

9. Composition selon l'une quelconque des revendications précédentes, **caractérisée par le fait qu'**elle renferme au moins un coupleur et/ou au moins un colorant direct.

10. Composition selon la revendication 9, **caractérisée par le fait que** le ou les coupleurs sont choisis parmi les métaphénylènediamines, les métaaminophénols, les métadiphénols et les coupleurs hétérocycliques, et leurs sels d'addition.

11. Composition selon l'une quelconque des revendications 9 à 10, **caractérisée par le fait que** le ou les coupleurs représentent de 0,0001 à 10 % en poids du poids total de la composition tinctoriale.

12. Composition selon l'une quelconque des revendications précédentes, **caractérisée par le fait que** les sels d'addition avec un acide sont choisis parmi les chlorhydrates, les bromhydrates, les sulfates et les taitrates, les lactates et les acétates et que les sels d'addition avec une base sont choisis parmi ceux obtenus avec la soude, la potasse, l'ammoniaque ou les amines.

13. Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 1 à 12, pendant un temps suffisant pour développer la coloration désirée, soit à l'air, soit à l'aide d'un agent oxydant, éventuellement en présence de catalyseurs d'oxydation.

14. Procédé selon la revendication 13, **caractérisé par le fait que** la coloration est révélée au seul contact de l'oxygène de l'air.

15. Procédé selon la revendication 13, **caractérisé par le fait que** l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

16. Procédé selon la revendication 13 ou 15, **caractérisé par le fait que** l'agent oxydant est choisi parmi le peroxyde d'hydrogène, le peroxyde d'urée, les bromates de métaux alcalins, les persels tels que les perborates et persulfates.

17. Dispositif à plusieurs compartiments, ou "kit" de teinture à plusieurs compartiments, dont un premier compartiment renferme une composition tinctoriale telle que définie à l'une quelconque des revendications 1 à 12 et un second compartiment renferme une composition oxydante.

18. Dérivés de pyrazolo-[1,5-a]-pyrimidine, leurs sels d'addition avec un acide ou une base et leurs formes tautomères lorsqu'il existe un équilibre tautomérique, ayant pour formule : 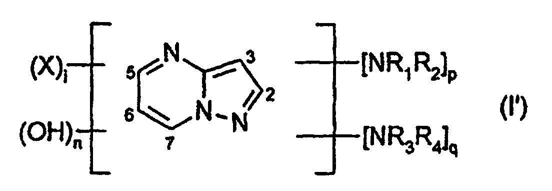 dans laquelle :
- R₁, R₂ R₃ et R₄ désignent , identiques ou différents un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical amino alkyle en C₁-C₄ (l'amine pouvant être protégée par un acétyle, un uréido, un sulfonyl), un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl- ou di-[hydroxy(C₁-C₄) alkyl]-amino alkyle en C₁-C₄ ;
- les radicaux X désignent , identiques ou différents, un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle aliphatique ou hétérocyclique à 5 ou 6 chaînons), un radical hydroxy(C₁-C₄)alkyl ou di-[hydroxy(C₁-C₄)alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;
- i vaut 0, 1, 2 ou 3 ;
- p vaut 0 ou 1 ;
- q vaut 0 ou 1 ;
- n vaut 0 ou 1 ;
sous réserve que :
- (i) la somme p + q est différente de 0 ;
- (ii) lorsque p + q est égal à 2 alors n vaut 0 et les groupes NR₁R₂ et NR₃R₄ occupent les positions (2,3) ; (5,6) ; (6,7) ; (3,5) ou (3,7) ;
- (iii) et lorsque p + q est égal à 1 alors n vaut 1 et le groupe NR₁R₂ (ou NR₃R₄) et le groupe OH occupent les positions (2,3) ; (5,6) ; (6,7) ; (3,5) ou (3,7) ; à l'exception des composés suivants :
- la pyrazolo-[1,5-a]-pyrimidine-6,7-diamine ;
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N 7, N 7-tétraméthyl pyrazolo-[1,5-a]-pyrimidine-3,7-diamine ;
- la 2,3-diméthyl pyrazolo-[1,5-a]-pyrimidine-6,7-diamine ;
- le 6-amino-5-méthyl-pyrazolo-[1,5-a]-pyrimidin-7-ol ;
- la 2,5-diméthyl 6-phényl-[1,5-a]-pyrimidine-3,7-diamine ;
- la 2,6-diméthyl 5-benzyl-[1,5-a]-pyrimidine-3,7-diamine ;
et de leurs sels d'addition.

19. Dérivés selon la revendication 18 choisis dans le groupe constitué par :
- la pyrazolo-[1,5-a]-pyrimidine-3,7-diamine ;
- la 2-méthyl pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol ;
- le 3-amino 5-méthyl pyrazolo-[1,5-a]-pyrimidin-7-ol ;
- le 3-amino pyrazolo-[1,5-a]-pyrimidin-5-ol ;
- le 2-(3-amino pyrazolo-[1,5-a]-pyrimidin-7-ylamino)-éthanol ;
- la 3-amino-7-β-hydroxyéthylamino-5-méthyl-pyrazolo-[1,5-a]-pyrimidine ;
- le 2-(7-amino pyrazolo-[1,5-a]-pyrimidin-3-ylamino)-éthanol ;
- le 2-[(3-amino-pyrazolo-[1,5-a]-pyrimidin-7-yl)-(2-hydroxyéthyl)-amino]-éthanol ;
- le 2-[(7-amino-pyrazolo-[1,5-a]-pyrimidin-3-yl)-(2-hydroxyéthyl)-amino]-éthanol ;
ainsi que leurs sels d'addition et leurs formes tautomères lorsqu'il existe un équilibre tautomérique.

## Claims

1. Composition for the oxidation dyeing of keratin fibres, and in particular human keratin fibres such as the hair, **characterized in that** it comprises, in a medium which is suitable for dyeing, at least one pyrazolo[1,5-a]pyrimidine derivative of formula (I) below as oxidation base and/or one of the addition salts thereof with an acid or with a base and/or one of the tautomeric forms thereof, when a tautomeric equilibrium exists: 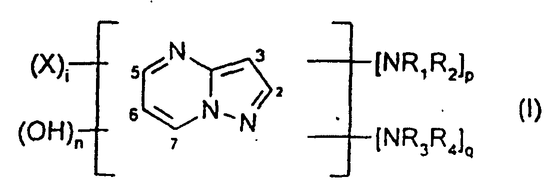 in which:
- R₁, R₂, R₃ and R₄, which may be identical or different, denote a hydrogen atom, a C₁-C₄ alkyl radical, an aryl radical, a C₁-C₄ hydroxyalkyl radical, a C₂-C₄ polyhydroxyalkyl radical, a (C₁-C₄)alkoxy(C₁-C₄)alkyl radical, a C₁-C₄ aminoalkyl radical (it being possible for the amine to be protected with an acetyl, a ureido or a sulphonyl), a (C₁-C₄)alkylamino(C₁-C₄)alkyl radical, a di[(C₁-C₄)alkyl]amino (C₁-C₄)alkyl radical (it being possible for the dialkyls to form a 5- or a 6-membered aliphatic or heterocyclic ring), or a hydroxy(C₁-C₄)alkylamino(C₁-C₄)alkyl or di[hydroxy(C₁-C₄)alkyl]amino(C₁-C₄)alkyl radical;
- the radicals X, which may be identical or different, denote a hydrogen atom, a C₁-C₄ alkyl radical, an aryl radical, a C₁-C₄ hydroxyalkyl radical, a C₂-C₄ polyhydroxyalkyl radical, a C₁-C₄ aminoalkyl radical, a (C₁-C₄)alkylamino(C₁-C₄)alkyl radical, a di[(C₁-C₄) alkyl]amino(C₁-C₄)alkyl radical (it being possible for the dialkyls to form a 5- or 6-membered aliphatic or heterocyclic ring), a hydroxy(C₁-C₄)alkylamino(C₁-C₄)alkyl radical, a di [hydroxy(C₁-C₄)alkyl]-amino(C₁-C₄)alkyl radical, an amino radical, a (C₁-C₄)alkylamino radical or a di[(C₁-C₄)alkyl]amino radical, a halogen atom, a carboxylic acid group or a sulphonic acid group;
- i is equal to 0, 1, 2 or 3;
- p is equal to 0 or 1;
- q is equal to 0 or 1;
- n is equal to 0 or 1;
with the proviso that:
- (i) the sum p + q is other than 0;
- (ii) when p + q is equal to 2, then n is equal to 0 and the groups NR₁R₂ and NR₃R₄ occupy positions (2,3); (5,6); (6,7); (3,5) or (3,7);
- (iii) and when p + q is equal to 1, then n is equal to 1 and the group NR₁R₂ (or NR₃R₄) and the OH group occupy positions (2,3); (5,6); (6,7); (3,5) or (3,7).

2. Composition according to Claim 1, **characterized in that** the pyrazolo[1,5-a]pyrimidine derivatives of formula (I) are chosen from:
- pyrazolo[1,5-a]pyrimidine-3,7-diamine;
- 2-methylpyrazolo[1,5-a]pyrimidine-3,7-diamine;
- 2,5-dimethylpyrazolo[1,5-a]pyrimidine-3,7-diamine;
- pyrazolo[1,5-a]pyrimidine-3,5-diamine;
- 2,7-dimethylpyrazolo[1,5-a]pyrimidine-3,5-diamine;
- 3-aminopyrazolo[1,5-a]pyrimidin-7-ol;
- 3-amino-5-methylpyrazolo[1,5-a]pyrimidin-7-ol;
- 3-aminopyrazolo[1,5-a]pyrimidin-5-ol;
- 2-(3-aminopyrazolo[1,5-a]pyrimidin-7-ylamino)ethanol;
- 3-amino-7-β-hydroxyethylamino-5-methylpyrazolo[1,5-a]pyrimidine;
- 2-(7-aminopyrazolo[1,5-a]pyrimidin-3-ylamino)ethanol;
- 2-[(3-aminopyrazolo[1,5-a]pyrimidin-7-yl)(2-hydroxyethyl)amino]ethanol;
- 2-[(7-aminopyrazolo[1,5-a]pyrimidin-3-yl)(2-hydroxyethyl)amino]ethanol;
- 5,6-dimethylpyrazolo[1,5-a]pyrimidine-3,7-diamine;
- 2,6-dimethylpyrazolo[1,5-a]pyrimidine-3,7-diamine;
- 2,5,N-7, N-7-tetramethylpyrazolo[1,5-a]pyrimidine-3,7-diamine;
and the addition salts thereof and the tautomeric forms thereof, when a tautomeric equilibrium exists.

3. Composition according to either of the preceding claims, **characterized in that** the pyrazolo[1,5-a]pyrimidine derivative(s) of formula (I) represent(s) from 0.0005 to 12% by weight relative to the total weight of the dye composition.

4. Composition according to Claim 3, **characterized in that** the pyrazolo[1,5-a]pyrimidine derivative(s) of formula (I) represent(s) from 0.005 to 6% by weight relative to the total weight of the dye composition.

5. Composition according to any one of the preceding claims, **characterized in that** the medium which is suitable for dyeing (or the support) consists of water or of a mixture of water and at least one organic solvent chosen from C₁-C₄ lower alkanols, glycerol, glycols and glycol ethers, aromatic alcohols, similar products and mixtures thereof.

6. Composition according to any one of the preceding claims, **characterized in that** it has a pH of between 3 and 12.

7. Composition according to any one of the preceding claims, **characterized in that** it contains at least one additional oxidation base chosen from para-phenylenediamines, bis(phenyl)alkylenediamines, para-aminophenols, ortho-aminophenols and heterocyclic bases other than the pyrazolo[1,5-a]pyrimidine derivatives of formula (I).

8. Composition according to Claim 7, **characterized in that** the additional oxidation base(s) represent(s) from 0.0005 to 12% by weight relative to the total weight of the dye composition.

9. Composition according to any one of the preceding claims, **characterized in that** it contains at least one coupler and/or at least one direct dye.

10. Composition according to Claim 9, **characterized in that** the coupler(s) is (are) chosen from meta-phenylenediamines, meta-aminophenols, meta-diphenols and heterocyclic couplers, and the addition salts thereof.

11. Composition according to either of Claims 9 and 10, **characterized in that** the coupler(s) represent(s) from 0.0001 to 10% by weight relative to the total weight of the dye composition.

12. Composition according to any one of the preceding claims, **characterized in that** the addition salts with an acid are chosen from the hydrochlorides, hydrobromides, sulphates, tartrates, lactates and acetates and **in that** the addition salts with a base are chosen from those obtained with sodium hydroxide, potassium hydroxide, aqueous ammonia or amines.

13. Process for dyeing keratin fibres, and in particular human keratin fibres such as the hair, **characterized in that** at least one dye composition as defined in any one of Claims 1 to 12 is applied to these fibres for a period which is sufficient to develop the desired coloration, either in air or using an oxidizing agent, optionally in the presence of oxidation catalysts.

14. Process according to Claim 13, **characterized in that** the coloration is developed merely by contact with atmospheric oxygen.

15. Process according to Claim 13, **characterized in that** the colour is developed at acidic, neutral or alkaline pH using an oxidizing agent which is added, only at the time of use, to the dye composition or which is present in an oxidizing composition that is applied simultaneously or sequentially in a separate manner.

16. Process according to Claim 13 or 15, **characterized in that** the oxidizing agent is chosen from hydrogen peroxide, urea peroxide, alkali metal bromates and persalts such as perborates and persulphates.

17. Multi-compartment device or multi-compartment dyeing "kit", a first compartment of which contains a dye composition as defined in any one of Claims 1 to 12 and a second compartment of which contains an oxidizing composition.

18. Pyrazolo [1,5-a]pyrimidine derivatives, the addition salts thereof with an acid or a base and the tautomeric forms thereof, when a tautomeric equilibrium exists, having the formula: 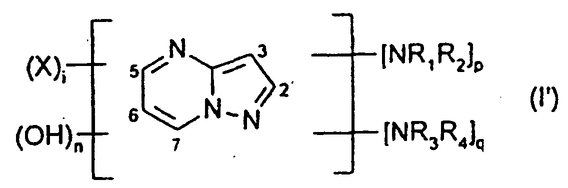 in which:
- R₁, R₂, R₃ and R₄, which may be identical or different, denote a hydrogen atom, a C₁-C₄ alkyl radical, an aryl radical, a C₁-C₄ hydroxyalkyl radical, a C₂-C₄ polyhydroxyalkyl radical, a (C₁-C₄)alkoxy(C₁-C₄)alkyl radical, a C₁-C₄ aminoalkyl radical (it being possible for the amine to be protected with an acetyl, a ureido or a sulphonyl), a (C₁-C₄)alkylamino(C₁-C₄)alkyl radical, a di[(C₁-C₄)alkyl]amino(C₁-C₄)alkyl radical (it being possible for the dialkyls to form a 5- or a 6-membered aliphatic or heterocyclic ring), or a hydroxy (C₁-C₄) alkylamino (C₁-C₄) alkyl radical or di [hydroxy (C₁-C₄) alkyl] amino (C₁-C₄) alkyl radical;
- the radicals X, which may be identical or different, denote a hydrogen atom, a C₁-C₄ alkyl radical, an aryl radical, a C₁-C₄ hydroxyalkyl radical, a C₂-C₄ polyhydroxyalkyl radical, a C₁-C₄ aminoalkyl radical, a (C₁ - C₄)alkylamino(C₁-C₄)alkyl radical, a di[(C₁-C₄) alkyl]amino(C₁-C₄)alkyl radical (it being possible for the dialkyls to form a 5- or 6-membered aliphatic or heterocyclic ring), a hydroxy(C₁-C₄)alkylamino(C₁-C₄)alkyl radical, a di[hydroxy(C₁-C₄)alkyl]amino(C₁-C₄)alkyl radical, an amino radical, a (C₁-C₄)alkylamino radical or a di[(C₁-C₄)alkyl]amino radical, a halogen atom, a carboxylic acid group or a sulphonic acid group;
- i is equal to 0, 1, 2 or 3;
- p is equal to 0 or 1;
- q is equal to 0 or 1;
- n is equal to 0 or 1;
with the proviso that:
- (i) the sum p + q is other than 0;
- (ii) when p + q is equal to 2, then n is equal to 0 and the groups NR₁R₂ and NR₃R₄ occupy positions (2,3); (5,6); (6,7); (3,5) or (3,7);
- (iii) and when p + q is equal to 1, then n is equal to 1 and the group NR₁R₂ (or NR₃R₄) and the OH group occupy positions (2,3); (5,6); (6,7); (3,5) or (3,7); except for the following compounds:
- pyrazolo[1,5-a]pyrimidine-6,7-diamine;
- 5,6-dimethylpyrazolo[1,5-a]pyrimidine-3,7-diamine;
- 2,6-dimethylpyrazolo[1,5-a]pyrimidine-3,7-diamine;
- 2,5-N7,N7-tetramethylpyrazolo[1,5-a]pyrimidine-3,7-diamine;
- 2,3-dimethylpyrazolo[1,5-a]pyrimidine-6,7-diamine;
- 6-amino-5-methylpyrazolo[1,5-a]pyrimidin-7-ol;
- 2,5-dimethyl-6-phenyl[1,5-a]pyrimidine-3,7-diamine;
- 2,6-dimethyl-5-benzyl[1,5-a]pyrimidine-3,7-diamine;
and the addition salts thereof.

19. Derivatives according to Claim 18, chosen from the group consisting of:
- pyrazolo[1,5-a]pyrimidine-3,7-diamine;
- 2-methylpyrazolo[1,5-a]pyrimidine-3,7-diamine;
- 2,5-dimethylpyrazolo[1,5-a]pyrimidine-3,7-diamine;
- pyrazolo[1,5-a]pyrimidine-3,5-diamine;
- 2,7-dimethylpyrazolo[1,5-a]pyrimidine-3,5-diamine;
- 3-aminopyrazolo[1,5-a]pyrimidin-7-ol;
- 3-amino-5-methylpyrazolo[1,5-a]pyrimidin-7-ol;
- 3-aminopyrazolo[1,5-a]pyrimidin-5-ol;
- 2-(3-aminopyrazolo[1,5-a]pyrimidin-7-ylamino)ethanol;
- 3-amino-7-β-hydroxyethylamino-5-methylpyrazolo[1,5-a] pyrimidine;
- 2-(7-aminopyrazolo[1,5-a]pyrimidin-3-ylamino)ethanol;
- 2-[(3-aminopyrazolo[1,5-a]pyrimidin-7-yl)-(2-hydroxyethyl)amino]ethanol;
- 2-[(7-aminopyrazolo[1,5-a]pyrimidin-3-yl)-(2-hydroxyethyl)amino]ethanol;
as well as the addition salts thereof and the tautomeric forms thereof when a tautomeric equilibrium exists.

## Patentansprüche

1. Zusammensetzung zum oxidativen Färben von Keratinfasern und insbesondere menschlichen Keratinfasern, wie dem Haar, **dadurch gekennzeichnet, daß** sie in einem zum Färben geeigneten Medium als Oxidationsbase mindestens ein Pyrazolo-[1,5-a]-pyrimidin der folgenden Formel (I) und/oder Additionssalze dieser Verbindungen mit einer Säure oder einer Base und/oder, falls ein tautomeres Gleichgewicht vorliegt, ihre Tautomeren enthält: 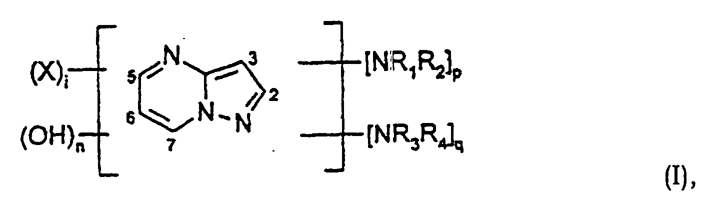 worin bedeuten:
- R₁, R₂, R₃ und R₄, die identisch oder voneinander verschieden sind, Wasserstoff, C₁₋₄-Alkyl, Aryl, C₁₋₄-Hydroxyalkyl, C₂₋₄-Polyhydroxyalkyl, C₁₋₄-Alkoxy-C₁₋₄-alkyl, C₁₋₄-Aminoalkyl (wobei die Aminogruppe mit Acetyl, Ureido oder Sulfonyl geschützt sein kann), C₁₋₄-Alkylamino-C₁₋₄-alkyl, Di-[C₁₋₄-alkyl]amino-C₁₋₄-alkyl (wobei die Dialkylgruppen einen aliphatischen oder heterocyclischen Ring mit 5 oder 6 Gliedern bilden können), Hydroxy-C₁₋₄alkylamino-C₁₋₄-alkyl oder Di-[hydroxy-C₁₋₄-alkyl]amino-C₁₋₄alkyl;
- die Gruppen X, die identisch oder voneinander verschieden sind, Wasserstoff, C₁₋₄-Alkyl, Aryl, C₁₋₄-Hydroxyalkyl, C₂₋₄-Polyhydroxyalkyl, C₁₋₄-Aminoalkyl, C₁₋₄-Alkylamino-C₁₋₄-alkyl, Di-[C₁₋₄alkyl]amino-C₁₋₄-alkyl (wobei die Dialkylgruppen einen aliphatischen oder heterocyclischen Ring mit 5 oder 6 Gliedern bilden können), Hydroxy-C₁₋₄-alkylamino-C₁₋₄-alkyl, Di-[Hydroxy-C₁₋₄alkyl]amino-C₁₋₄-alkyl, Amino, C₁₋₄-Alkylamino, Di-[C₁₋₄alkyl]amino, Halogen, Carboxy oder eine Sulfonsäuregruppe;
- i 0, 1, 2 oder 3;
- p 0 oder 1:
- q 0 oder 1;
- n 0 oder 1;
mit der Maßgabe, daß
(i) die Summe p + q von Null verschieden ist;
(ii) n Null bedeutet und die Gruppen NR₁R₂ und NR₃R₄ die Stellungen (2,3); (5,6); (6,7); (3,5) oder (3,7) einnehmen, wenn p + q 2 ist;
(iii) n 1 bedeutet und die Gruppe NR₁R₂ (oder NR₃R₄) und die OH-Gruppe die Stellungen (2,3); (5,6); (6,7); (3,5) oder (3,7) einnehmen, wenn p + q 1 ist.

2. Zusammensetzung nach Anspruch 1, **dadurch gekennzeichnet, daß** die Pyrazolo-[1,5-a]-pyrimidine ausgewählt sind unter:
- Pyrazolo-[1,5-a]-pyrimidin-3,7-diamin,
- 2-Methyl-pyrazolo-[1,5-a]-pyrimidin-3,7-diamin,
- 2,5-Dimethyl-pyrazolo-[1,5-a]-pyrimidin-3,7-diamin,
- Pyrazolo-[1,5-a]-pyrimidin-3,5-diamin,
- 2,7-Dimethyl-pyrazolo-[1,5-a]-pyrimidin-3,5-diamin,
- 3-Amino-pyrazolo-[1,5-a]-pyrimidin-7-ol,
- 3-Amino-5-methyl-pyrazolo-[1,5-a]-pyrimidin-7-ol,
- 3-Amino-pyrazolo-[1,5-a]-pyrimidin-5-ol,
- 2-(3-Amino-pyrazolo-[1,5-a]-pyrimidin-7-ylamino)-ethanol,
- 3-Amino-7-β-hydroxyethylamino-5-methyl-pyrazolo-[1,5-a]-pyrimidin,
- 2-(7-Amino-pyrazolo-[1,5-a]-pyrimidin-3-ylamino)-ethanol,
- 2-[(3-Amino-pyrazolo-[1,5-a]-pyrimidin-7-yl)-(2-hydroxyethyl)amino]-ethanol,
- 2-[(7-Amino-pyrazolo-[1,5-a]-pyrimidin-3-yl)-(2-hydroxyethyl)amino]-ethanol,
- 5,6-Dimethyl-pyrazolo-[1,5-a]-pyrimidin-3,7-diamin,
- 2,6-Dimethyl-pyrazolo-[1,5-a]-pyrimidin-3,7-diamin,
- 2,5,N7,N7-Tetramethyl-pyrazolo-[1,5-a]-pyrimidin-3,7-diamin
- und deren Additionssalze und deren tautomeren Formen, wenn tautomere Formen im Gleichgewicht vorliegen.

3. Zusammensetzung nach einem der Ansprüche 1 oder 2, **dadurch gekennzeichnet, daß** das oder die Pyrazolo-[1,5-a]-pyrimidine der Formel (I) 0,0005 bis 12 Gew.-% des Gesamtgewichts der Farbmittelzusammensetzung ausmachen.

4. Zusammensetzung nach Anspruch 3, **dadurch gekennzeichnet, daß** das oder die Pyrazolo-[1,5-a]-pyrimidine der Formel (I) 0,005 bis 6 Gew.-% des Gesamtgewichts der Farbmittelzusammensetzung ausmachen.

5. Zusammensetzung nach einem der vorhergehenden Ansprüche, **dadurch gekennzeichnet, daß** das zum Färben geeignete Medium (oder der Träger) aus Wasser oder einem Gemisch von Wasser und mindestens einem organischen Lösungsmittel besteht, das unter den niederen C₁₋₄-Alkanolen, Glycerin, Glykolen und Glykolethern, aromatischen Alkoholen, analogen Produkten und deren Gemischen ausgewählt ist.

6. Zusammensetzung nach einem der vorhergehenden Ansprüche, **dadurch gekennzeichnet, daß** sie einen pH-Wert im Bereich von 3 bis 12 aufweist.

7. Zusammensetzung nach einem der vorhergehenden Ansprüche, **dadurch gekennzeichnet, daß** sie mindestens eine zusätzliche Oxidationsbase enthält, die unter den p-Phenylendiaminen, Bisphenylalkylendiaminen, p-Aminophenolen, o-Aminophenolen und heterocyclischen Basen, die von den Pyrazolo-[1,5-a]-pyrimidinen der Formel (I) verschieden sind, ausgewählt ist.

8. Zusammensetzung nach Anspruch 7, **dadurch gekennzeichnet, daß** die zusätzliche(n) Oxidationsbase(n) 0,0005 bis 12 Gew.-% des Gesamtgewichts der Farbmittelzusammensetzung ausmachen.

9. Zusammensetzung nach einem der vorhergehenden Ansprüche, **dadurch gekennzeichnet, daß** sie mindestens einen Kuppler und/oder mindestens einen Direktfarbstoff enthält.

10. Zusammensetzung nach Anspruch 9, **dadurch gekennzeichnet, daß** der oder die Kuppler unter den m-Phenylendiaminen, m-Aminophenolen, m-Dihydroxybenzolen und den heterocyclischen Kupplern und deren Additionssalzen ausgewählt sind.

11. Zusammensetzung nach einem der Ansprüche 9 bis 10, **dadurch gekennzeichnet, daß** der oder die Kuppler 0,0001 bis 10 Gew.-% des Gesamtgewichts der Farbmittelzusammensetzung ausmachen.

12. Zusammensetzung nach einem der vorhergehenden Ansprüche, **dadurch gekennzeichnet, daß** die Additionssalze mit einer Säure unter den Hydrochloriden, Hydrobromiden, Sulfaten, Tartraten, Lactaten und Acetaten und die Additionsalze mit einer Base unter den Salzen ausgewählt sind, die mit Natriumhydroxid, Kaliumhydroxid, Ammoniak oder Aminen erhalten werden.

13. Verfahren zum Färben von Keratinfasern und insbesondere menschlichen Keratinfasern, **dadurch gekennzeichnet, daß** mindestens eine Farbmittelzusammensetzung nach einem der Ansprüche 1 bis 12 während einer Zeitspanne auf die Faser aufgebracht wird, die ausreichend ist, um die gewünschte Färbung gegebenenfalls in Gegenwart von Oxidationskatalysatoren entweder mit Luftsauerstoff oder einem Oxidationsmittel zu entwickeln.

14. Verfahren nach Anspruch 13, **dadurch gekennzeichnet, daß** die Farbe nur mit Luftsauerstoff entwickelt wird

15. Verfahren nach Anspruch 13, **dadurch gekennzeichnet, daß** die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgetragen wird.

16. Verfahren nach Anspruch 13 oder 15, **dadurch gekennzeichnet, daß** das Oxidationsmittel unter Wasserstoffperoxid, Harnstoffperoxid, Alkalimetallbromaten und Salzen von Persäuren, wie Perboraten und Persulfaten, ausgewählt ist.

17. Vorrichtung mit mehreren Abteilungen oder "Kit" zum Färben mit mehreren Abteilungen, wobei eine erste Abteilung eine Farbmittelzusammensetzung nach einem der Ansprüche 1 bis 12 und eine zweite Abteilung eine oxidierende Zusammensetzung enthält.

18. Pyrazolo-[1,5-a]-pyrimidine, die Additionssalze dieser Verbindungen mit einer Säure oder einer Base und, falls ein tautomeres Gleichgewicht vorliegt, ihre Tautomeren der folgenden Formel (I'): 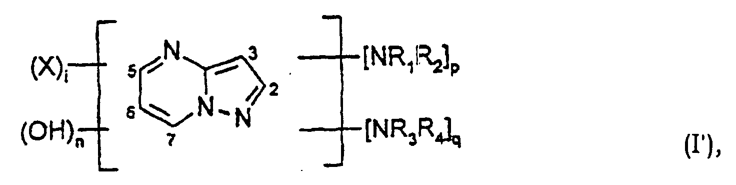 worin bedeuten:
- R₁, R₂, R₃ und R₄, die identisch oder voneinander verschieden sind, Wasserstoff, C₁₋₄-Alkyl, Aryl, C₁₋₄-Hydroxyalkyl, C₂₋₄-Polyhydroxyalkyl, C₁₋₄-Alkoxy-C₁₋₄-alkyl, C₁₋₄-Aminoalkyl (wobei die Aminogruppe mit Acetyl, Ureido oder Sulfonyl geschützt sein kann), C₁₋₄-Alkylamino-C₁₋₄-alkyl, Di-[C₁₋₄-alkyl]amino-C₁₋₄-alkyl (wobei die Dialkylgruppen einen aliphatischen oder heterocyclischen Ring mit 5 oder 6 Gliedern bilden können), Hydroxy-C₁₋₄-alkylamino-C₁₋₄-alkyl oder Di-[hydroxy-C₁₋₄-alkyl]amino-C₁₋₄alkyl;
- die Gruppen X, die identisch oder voneinander verschieden sind, Wasserstoff, C₁₋₄-Alkyl, Aryl, C₁₋₄-Hydroxyalkyl, C₂₋₄-Polyhydroxyalkyl, C₁₋₄-Aminoalkyl, C₁₋₄-Alkylamino-C₁₋₄-alkyl, Di-[C₁₋₄alkyl]amino-C₁₋₄-alkyl (wobei die Dialkylgruppen einen aliphatischen oder heterocyclischen Ring mit 5 oder 6 Gliedern bilden können), Hydroxy-C₁₋₄-alkylamino-C₁₋₄-alkyl, Di-[Hydroxy-C₁₋₄alkyl]amino-C₁₋₄-alkyl, Amino, C₁₋₄-Alkylamino, Di-[C₁₋₄alkyl]amino, Halogen, Carboxy oder eine Sulfonsäuregruppe;
- i 0, 1, 2 oder 3;
- p 0 oder 1:
- q 0 oder 1;
- n 0 oder 1;
mit der Maßgabe, daß
(iv) die Summe p + q von Null verschieden ist;
(v) n Null bedeutet und die Gruppen NR₁R₂ und NR₃R₄ die Stellungen (2,3); (5,6); (6,7); (3,5) oder (3,7) einnehmen, wenn p + q 2 ist;
(vi) n 1 bedeutet und die Gruppe NR₁R₂ (oder NR₃R₄) und die OH-Gruppe die Stellungen (2,3); (5,6); (6,7); (3,5) oder (3,7) einnehmen, wenn p + q 1 ist.
wobei die folgenden Verbindungen ausgenommen sind:
- Pyrazolo-[1,5-a]-pyrimidin-6,7-diamin;
- 5,6-Dimethyl-pyrazolo-[1,5-a]-pyrimidin-3,7-diamin;
- 2,6-Dimethyl-pyrazolo-[1,5-a]-pyrimidin-3,7-diamin;
- 2,5,N7,N7-Tetramethyl-pyrazolo-[1,5-a]-pyrimidin-3,7-diamin;
- 2,3-Dimethyl-pyrazolo-[1,5-a]-pyrimidin-6,7-diamin;
- 6-Amino-5-methyl-pyrazolo-[1,5-a]-pyrimidin-7-ol;
- 2,5-Dimethyl-6-phenyl-pyrazolo-[1,5-a]-pyrimidin-3,7-diamin;
- 2,6-Dimethyl-5-benzyl-pyrazolo-[1,5-a]-pyrimidin-3,7-diamin; und
- deren Additionssalze.

19. Derivate nach Anspruch 18, die ausgewählt sind unter:
- Pyrazolo-[1,5-a]-pyrimidin-3,7-diamin,
- 2-Methyl-pyrazolo-[1,5-a]-pyrimidin-3,7-diamin,
- 2,5-Dimethyl-pyrazolo-[1,5-a]-pyrimidin-3,7-diamin,
- Pyrazolo-[1,5-a]-pyrimidin-3,5-diamin,
- 2,7-Dimethyl-pyrazolo-[1,5-a]-pyrimidin-3,5-diamin,
- 3-Amino-pyrazolo-[1,5-a]-pyrimidin-7-ol,
- 3-Amino-5-methyl-pyrazolo-[1,5-a]-pyrimidin-7-ol,
- 3-Amino-pyrazolo-[1,5-a]-pyrimidin-5-ol,
- 2-(3-Amino-pyrazolo-[1,5-a]-pyrimidin-7-ylamino)-ethanol,
- 3-Amino-7-β-hydroxyethylamino-5-methyl-pyrazolo-[1,5-a]-pyrimidin,
- 2-(7-Amino-pyrazolo-[1,5-a]-pyrimidin-3-ylamino)-ethanol,
- 2-[(3-Amino-pyrazolo-[1,5-a]-pyrimidin-7-yl)-(2-hydroxyethyl)amino]-ethanol,
- 2-[(7-Amino-pyrazolo-[1,5-a]-pyrimidin-3-yl)-(2-hydroxyethyl)amino]-ethanol,
- und deren Additionssalze und deren tautomeren Formen, wenn tautomere Formen im Gleichgewicht vorliegen.
